# FASCICULE DE BREVET EUROPEEN

(11) **EP 0 379 412 B1**
(45) Date de publication et mention de la délivrance du brevet: **01.12.1993**
(21) Numéro de dépôt: 90400104.7
(22) Date de dépôt: 15.01.1990
(51) Int. Cl.: C07D 471/04, A61K 31/495, A61K 31/55

(54) **Dérivés de benzonaphtyridine-1,8 leur préparation et les compositions qui les contiennent**
1,8-Benzonaphthyridinderivate, ihre Herstellung und diese enthaltende Zusammensetzungen
1,8-Benzonaphthyridine derivatives, their preparation and compositions containing them

(30) Priorité: 16.01.1989 FR 8900430; 28.07.1989 FR 8910218
(43) Date de publication de la demande: 25.07.1990
(73) Titulaire: Société anonyme dite: LABORATOIRE ROGER BELLON, 92201 Neuilly-sur-Seine (FR)
(72) Inventeur: Antoine, Michel, F-75013 Paris (FR); Barreau, Michel, F-91230 Montgeron (FR); Desconclois, Jean-François, F-75012 Paris (FR); Girard, Philippe, F-91290 Arpajon (FR); Picaut, Guy, F-94550 Chevilly Larue (FR)
(74) Mandataire: Savina, Jacques

(56) Documents cités:
- EP-A- 0 009 425
- CHEMICAL ABSTRACTS, vol. 61, no. 9, 26 octobre 1964, résumé no. 10666g, Columbus, Ohio, US; A.K. MALLAMS: "New synthesis of dibenzo[b,g][1,8]naphthyridines"
- CHEMISCHE BERICHTE, vol. 106, no. 11, 1973, pages 3533-3539; J. LEHMANN et al.: "Heterocyclische Beta-Enamino-ester, X. Zur Reaktivität von 2-Amino-3-chinolincarbonsäure-äthylester gegenüber Isocyanaten, Isothiocyanaten und Lactimäthern"

## Description

La présente invention concerne des dérivés de la benzo[b]naphtyridine-1,8 de formule générale : 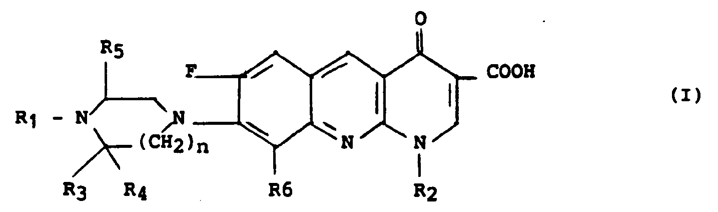
leurs sels, leur préparation et les compositions qui les contiennent.

Dans les brevets US 4 229 456 et 4 133 885 ont été décrits des dérivés de naphtyridine de structure : 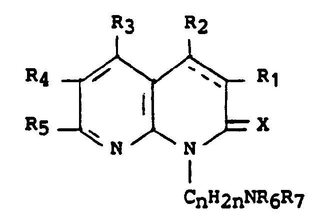
dans laquelle X peut être l'oxygène et deux des radicaux R₁ à R₅ adjacents peuvent former un cycle benzénique.

Ces produits sont utiles comme inhibiteurs des sécrétions gastriques acides.

La demande de brevet DE 3 302 126 décrit des hypotenseurs de formule générale : 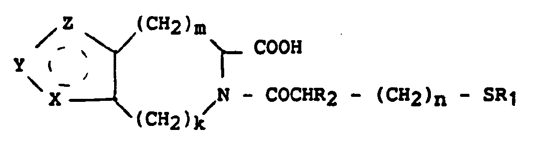
dans laquelle les radicaux X, Y et Z peuvent être O ou un radical NR₄ ou CR₅=CR₅ dans lequel les R₅ peuvent former un cycle benzénique.

Il a été trouvé que les produits de formule générale (I) dans laquelle :
- R₁ représente un atome d'hydrogène ou un radical alcoyle ou hydroxyalcoyle,
- R₂ représente un atome d'hydrogène ou un radical alcoyle, fluoroalcoyle, cycloalcoyle, alcoyloxy ou alcoylamino,
- R₃ représente un radical alcoyle, et
- R₄ et R₅ sont différents entre eux et représentent un atome d'hydrogène ou un radical alcoyle, ou bien
- R₃ représente un atome d'hydrogène ou un radical alcoyle ou cycloalcoyle et R₄ et R₅ sont des atomes d'hydrogène,
- R₆ représente un atome d'hydrogène ou de fluor et
- n égale 1 ou 2.

et dont les radicaux alcoyle sont droits ou ramifiés et contiennent 1 à 4 atomes de carbone et les radicaux cycloalcoyle contiennent 3 à 6 atomes de carbone, ainsi que leurs sels, et le cas échéant leurs isomères, manifestent une activité antibactérienne particulièrement intéressante.

Les produits de formule générale (I) peuvent exister à l'état de forme hydratée, il est entendu que ces hydrates entrent aussi dans le cadre de la présente invention.

Selon l'invention, les produits de formule générale (I) peuvent être obtenus par substitution d'une pipérazine de formule générale : 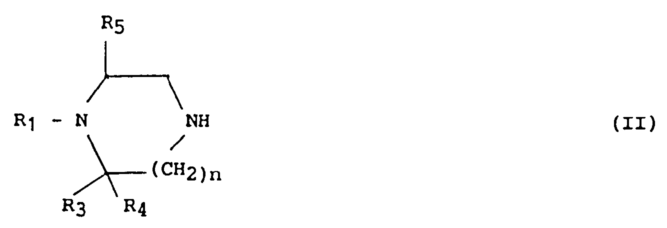
dans laquelle R₁, R₃, R₄, R₅ et n sont définis comme précédemment, sur une benzo[b]naphtyridine-1,8 de formule générale : 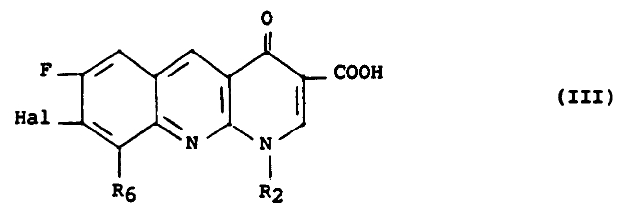
dans laquelle R₂ est défini comme précédemment et Hal est un atome de fluor, de chlore ou de brome, si R₆ est hydrogène, ou Hal et R₆ sont simultanément des atomes de fluor, suivie éventuellement, si R₁ est un atome d'hydrogène et si l'on veut obtenir un dérivé de benzo[b]naphtyridine-1,8 dans laquelle R₁ est méthyle, de la transformation du produit obtenu en une (méthyl-4 pipérazinyl-1)-8 benzo[b]naphtyridine.

L'action du dérivé de la pipérazine de formule générale (II) s'effectue généralement en présence d'un excès de ce dérivé comme accepteur d'acide ou en présence d'un accepteur d'acide organique ou minéral dans des solvants organiques convenables. Il est possible d'opérer avec ou sans solvant, à une température comprise entre 30 et 120°C. Lorsque l'on opère en présence d'un solvant, la réaction s'effectue avantageusement dans des solvants tels que la pyridine, le diméthylformamide, le diméthylsulfoxyde ou l'acétonitrile.

Il est entendu que, dans le cas où le symbole R₂ du produit de formule générale (III) est un atome d'hydrogène, il est préférable de protéger préalablement ce produit. La protection et l'élimination du radical protecteur après la réaction, s'effectuent selon les méthodes habituelles.

La protection peut être réalisée par tout groupement compatible et dont la mise en oeuvre et l'élimination n'altère pas le reste de la molécule. Notamment, on opère selon les méthodes décrites par T.W. GREENE, Protective Groups in Organic Synthesis, A. Wiley-Interscience Publication (1981), ou par Mc OMIE, Protective Groups in Organic Chemistry, Plenum Press (1973).

A titre d'exemple, les groupements protecteurs peuvent être choisis parmi les radicaux triméthylsilyle, méthoxyméthyle, éthoxyméthyle, benzhydryle, trityle, tétrahydropyrannyle, formyle, acétyle, chloracétyle, trichloracétyle, trifluoracétyle, méthoxycarbonyle, éthoxycarbonyle, t.butoxycarbonyle, trichloréthoxycarbonyle.

Le cas échéant, l'opération subséquente de méthylation du radical pipérazinyle s'effectue avantageusement par action du formol en présence d'acide formique. On opère généralement en milieu aqueux, à une température comprise entre 90 et 100°C.

Selon l'invention, les dérivés de benzo[b]naphtyridine-1,8 de formule générale (I) peuvent être aussi obtenus à partir de l'ester correspondant de formule générale : 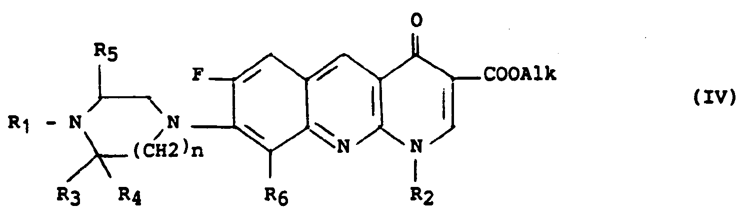
dans laquelle R₁, R₂, R₃, R₄, R₅, R₆ et n sont définis comme précédemment ou R₂ représente un radical alcoylamino protégé et Alk représente un radical alcoyle contenant 1 à 4 atomes de carbone en chaîne droite ou ramifiée, par toute méthode connue pour obtenir un acide à partir d'un ester sans toucher au reste de la molécule, suivie le cas échéant de l'élimination du groupement protecteur du radical alcoylamino, et/ou si l'on a obtenu un produit de formule générale (I) dans lequel R₁ est un atome d'hydrogène et si l'on veut obtenir le produit correspondant pour lequel R₁ est méthyle, de la transformation du produit obtenu en une (méthyl-4 pipérazinyl-1)-8 benzo[b]naphtyridine.

La préparation de l'acide à partir de l'ester s'effectue généralement par hydrolyse acide. On opère avantageusement dans un mélange acide acétique - acide chlorhydrique, dans l'acide sulfurique ou dans l'acide méthanesulfonique à une température comprise entre 20 et 100°C. Il est également possible d'opérer par saponification en présence de potasse ou de soude, en milieu hydro-alcoolique, à une température comprise entre 20 et 80°C.

Le cas échéant, la méthylation du radical pipérazinyle s'effectue, comme décrit précédemment.

Lorsque R₂ représente un radical alcoylamino protégé, le radical protecteur peut être tout groupement protecteur d'amino compatible avec la molécule. Il est particulièrement avantageux de choisir un radical protecteur qui peut être éliminé simultanément à l'hydrolyse de l'ester (par exemple le radical formyle) ;

Le dérivé de la benzo[b]napthyridine-1,8 de formule générale (III) peut être obtenu à partir de l'ester correspondant de formule générale : 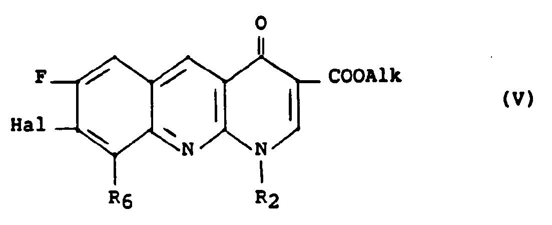
dans laquelle Hal, R₆ et Alk sont définis comme précédemment, et R₂ est défini comme précédemment ou représente un radical alcoylamino protégé, par toute méthode connue pour obtenir un acide à partir d'un ester, sans toucher au reste de la molécule, suivie éventuellement de l'élimination du groupement protecteur du radical alcoylamino.

On opère notamment dans les conditions décrites précédemment pour obtenir un produit de formule générale (I) à partir d'un ester de formule générale (IV).

L'ester dérivé de la benzo[b]naphtyridine-1,8 de formule générale (V) peut être préparé par action de l'amino-3 triazine-1,2,4 pour obtenir un produit pour lequel R₂ est un atome d'hydrogène ou par action d'un produit de formule générale :

R₂ - NH₂ (VI)

dans laquelle R₂ est alcoyle, fluoroalcoyle, cycloalcoyle, alcoyloxy ou alcoylamino éventuellement protégé sur un dérivé de la quinoléine de formule générale : 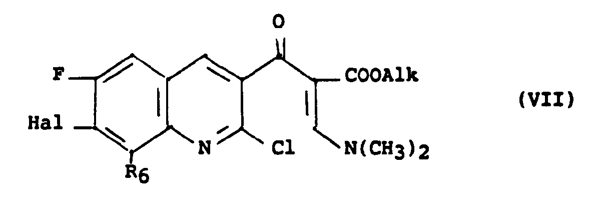
dans laquelle R₆, Hal et Alk sont définis comme précédemment, suivie de la cyclisation par action d'un agent accepteur d'acide.

Généralement, la réaction de l'amino-3 triazine-1,2,4 ou du produit de formule générale (VI) est mise en oeuvre dans un solvant organique comme un alcool (éthanol, méthanol par exemple) ou un solvant chloré (trichlorométhane par exemple), à une température comprise entre 10 et 25°C.

La cyclisation s'effectue dans un alcool à chaîne droite ou ramifiée contenant 1 à 4 atomes de carbone, à une température comprise entre 20°C et la température de reflux du mélange réactionnel.

L'agent accepteur d'acide peut être notamment choisi parmi les bases azotées (triéthylamine par exemple ou un excès de l'amine employée) ou le diaza-1,8-bicyclo[5.4.0] undécène-7

Le dérivé de la quinoléine de formule générale (VII) peut être obtenu à partir du cétoester de formule générale : 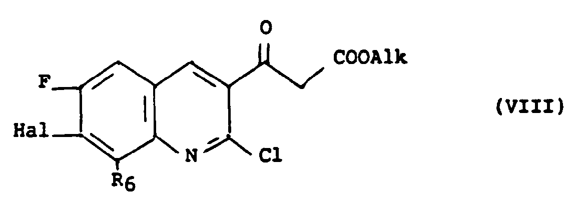
dans laquelle R₆, Hal et Alk sont définis comme précédemment, par action d'un NN-diméthylformamide acétal de formule générale :

(CH₃)₂N - CH(OAlk₁)₂ (IX)

dans laquelle Alk₁ est un radical alcoyle contenant 1 à 4 atomes de carbone en chaîne droite ou ramifiée.

La réaction s'effectue généralement dans un solvant organique tel qu'un ester (acétate d'éthyle par exemple) à une température comprise entre 60 et 75°C.

Le cétoester de formule générale (VIII) pour lequel R₆ est un atome d'hydrogène peut être obtenu à partir de l'acide dichloro-2,7 fluoro-6 quinoléine carboxylique-3 ou de l'acide chloro-2 difluoro-6,7 quinoléine carboxylique-3 comme décrit ci-après aux exemples 1 et 23 ou à partir de l'acide bromo-7 chloro-2 fluoro-6 quinoléine carboxylique-3 par analogie avec cette méthode. Dans ce cas, la bromo-3 fluoro-4 aniline utilisée comme produit de départ, peut être préparée selon la méthode décrite par W.B. Austin et coll., J. Org. Chem., 46(11), 2280 (1981).

Le cétoester de formule générale (VIII) pour lequel R₆ est un atome de fluor peut être obtenu à partir de l'acide chloro-2 trifluoro-6,7,8 quinoléine carboxylique-3 comme décrit ci-après à l'exemple 25.

Le dérivé de benzo[b]naphtyridine-1,8 de formule générale (IV) peut être obtenu à partir de la benzo[b]naphtyridine de formule générale (V), par substitution d'un dérivé de la pipérazine de formule générale (II).

On opère avantageusement dans les conditions décrites précédemment pour obtenir un produit de formule générale (I) à partir d'une pipérazine de formule générale (II) et d'une benzo[b]naphtyridine-1,8 de formule générale (III).

Les dérivés de la pipérazine de formule générale (II) peuvent être obtenus selon ou par analogie avec les méthodes décrites par :
- S. HARRY et coll., J. Am. Chem. Soc., 75, 4949 (1963),
- G. CIGNARELLA, J. Med. Chem., 7, 241 (1964) ou J. Het. Chem., 11, 985 (1974),
- J.R. PIPER, J. Org. Chem., 28, 981 (1963),
- ISCHIGURO, MATSUMARA, J. Pharm. Soc. Japan, 79, 153 et 302 (1959).

Selon l'invention, lorsque R₃ et R₄ sont différents, les dérivés de la benzo[b]naphtyridine de formule générale (I) présentent des formes isomères. Il est entendu que ces isomères et leurs mélanges entrent dans le cadre de la présente invention.

Lorsque l'on veut obtenir les isomères des dérivés de benzonaphtyridine de formule générale (I), on effectue la séparation des formes isomères des pipérazines de formule générale (II), par toute méthode connue et compatible avec la molécule. A titre d'exemple, la séparation s'effecte par acylation au moyen d'un acide chiral, séparation des isomères par chromatographie liquide hautes performances, puis désacylation par hydrolyse acide.

Les nouveaux produits selon la présente invention ainsi que leurs intermédiaires de synthèse peuvent être éventuellement purifiés par des méthodes physiques telles que la cristallisation ou la chromatographie.

Les produits selon la présente invention peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Les nouveaux produits selon l'invention peuvent être également transformés en sels d'addition avec les acides. Les produits de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, p.toluènesulfonates).

Les nouveaux dérivés de benzo[b]naphtyridine-1,8 de formule générale (I) selon la présente invention et leurs sels pharmaceutiquement acceptables présentent des propriétés antibactériennes particulièrement intéressantes. Ils manifestent une activité remarquable in vitro et in vivo sur les germes gram-positifs et d'une manière générale sur les germes responsables de la plupart des infections des voies aériennes hautes et basses.

In vitro, les produits de formule générale (I) se sont montrés actifs à une concentration comprise entre 0,12 et 50 µg/cm3 sur staphylococcus aureus IP 8203.

In vivo, les produits de formule générale (I) se sont montrés actifs sur les infections expérimentales de la souris à staphylococcus aureus IP 8203 à des doses comprises entre 2 et 150 mg/kg par voie orale ou sous cutanée.

Un autre intérêt des produits selon l'invention est leur faible toxicité. Leur DL₅₀ est généralement supérieure à 500 mg/kg par voie sous cutanée chez la souris.

D'un intérêt particulier sont les produits de formule générale (I) pour lesquels
- R₁ représente un atome d'hydrogène ou un radical alcoyle contenant 1 ou 2 atomes de carbone, éventuellement substitué par un radical hydroxy,
- R₂ représente un atome d'hydrogène, un radical alcoyle contenant 1 à 4 atomes de carbone et chaine droite ou ramifiée éventuellement substitué par un atome de fluor,
- R₃ représente un radical méthyle et
- R₄ et R₅ qui sont différents représentent un atome d'hydrogène ou un radical méthyle, ou bien
- R₃ est un atome d'hydrogène ou un radical méthyle et R₄ et R₅ représentent des atomes d'hydrogène,
- R₆ représente un atome d'hydrogène ou de fluor et
- n égale 1 ou 2.

Et parmi ces produits, plus spécialement intéressants sont :
- l'acide fluoro-7 méthyl-1 (méthyl-3 pipérazinyl-1)-8 oxo-4 dihydro-1,4 benzo[b]naphtyridine-1,8 carboxylique-3
- l'acide éthyl-1 fluoro-7 (pipérazinyl-1)-8 oxo-4 dihydro-1,4 benzo[b]naphtyridine-1,8 carboxylique-3
- l'acide cyclopropyl-1 fluoro-7 (pipérazinyl-1)-8 oxo-4 dihydro-1,4 benzo[b]naphtyridine-1,8 carboxylique-3
- l'acide difluoro-7,9 méthyl-1 (méthyl-4 pipérazinyl-1)-8 oxo-4 dihydro-1,4 benzo[b]naphtyridine-1,8 carboxylique-3
- l'acide (diméthyl-3,4 pipérazinyl-1)-8 éthyl-1 fluoro-7 oxo-4 dihydro-1,4 benzo[b]naphtyridine-1,8 carboxylique-3

Les exemples suivants donnés à titre non limitatif illustrent la présente invention.

### EXEMPLE 1

Une suspension de 3,5 g d'acide chloro-8 fluoro-7 méthyl-1 oxo-4 dihydro-1,4 benzo[b]naphtyridine-1,8 carboxylique-3 et de 4,6 g de méthyl-2 pipérazine dans 40 cm3 de pyridine est chauffée à température voisine de 115°C sous agitation pendant 13 heures. Le mélange réactionnel est concentré à sec sous pression réduite (20 kPa) à 60°C. Le résidu est repris 2 fois par 30 cm3 d'éthanol et concentré sous pression réduite dans les conditions ci-dessus. Le solide obtenu est repris par 60 cm3 d'eau et 10 cm3 de potasse aqueuse à 30 %. La phase aqueuse est lavée par 2 fois 100 cm3 de trichlorométhane, additionnée de 10,28 g d'acide méthanesulfonique lavée de nouveau par 2 fois 100 cm3 de trichlorométhane. On ajoute 10 cm3 de potasse aqueuse à 30 %. Le précipité formé est essoré, lavé par 3 fois 10 cm3 d'eau et 2 fois 10 cm3 d'éthanol. On obtient 2,7 g d'acide fluoro-7 méthyl-1 (méthyl-3 pipérazinyl-1)-8 oxo-4 dihydro-1,4 benzo[b]naphtyridine-1,8 carboxylique-3 sous forme de solide jaune fondant à 360-363°C.

L'acide chloro-8 fluoro-7 méthyl-1 oxo-4 dihydro-1,4 benzo[b]naphtyridine-1,8 carboxylique-3 peut être préparé de la manière suivante:

Une suspension de 15 g de chloro-8 éthoxycarbonyl-3 fluoro-7 méthyl-1 oxo-4 dihydro-1,4 benzo[b]naphtyridine-1,8 dans 150 cm3 d'acide acétique et 150 cm3 d'acide chlorhydrique en solution aqueuse à 17,5 % est chauffée à une température voisine de 100°C sous agitation pendant 4 heures. Après refroidissement à température voisine de 20°C, le produit est essoré, lavé par 2 fois 150 cm3 d'éthanol puis 2 fois 100 cm3 d'éther éthylique. On obtient 12,7 g d'acide chloro-8 fluoro-7 méthyl-1 oxo-4 dihydro-1,4 benzo[b]naphtyridine-1,8 carboxylique-3 sous forme d'un solide beige se sublimant à 400-405°C qui est utilisé sans autre purification pour les étapes ultérieures.

La chloro-8 éthoxycarbonyl-3 fluoro-7 méthyl-1 oxo-4 dihydro-1,4 benzo[b]naphtyridine-1,8 est préparée de la manière suivante :

Dans une suspension sous agitation de 19,3 g de (dichloro-2,7 fluoro-6 quinoléinecarbonyle-3)-2 diméthylamino-3 acrylate d'éthyle dans 250 cm3 d'éthanol, maintenue entre 10 et 15°C, on fait barboter de la méthylamine jusqu'à absorption de 16 g de gaz. On laisse la température remonter à environ 20°C, ajoute 0,8 g de diaza-1,8-bicyclo[5.4.0.]undécène-7 (DBU) et chauffe à une température voisine de 75°C pendant 2 heures. Après refroidissement à environ 20°C, le produit est essoré, lavé par 2 fois 150 cm3 d'éthanol et 2 fois 100 cm3 d'éther éthylique. On obtient 15 g de chloro-8 éthoxycarbonyl-3 fluoro-7 méthyl-1 oxo-4 dihydro-1,4 benzo[b]naphtyridine-1,8 sous forme d'un solide jaune fondant à 360-362°C qui est utilisé sans autre purification pour les étapes ultérieures.

Le (dichloro-2,7 fluoro-6 quinoléinecarbonyle-3)-2 diméthylamino-3 acrylate d'éthyle est préparé de la manière suivante :

Une suspension de 16,5 g de (dichloro-2,7 fluoro-6 quinolyl-3)-3 oxo-3 propionate d'éthyle dans 160 cm3 d'acétate d'éthyle et 19 cm3 de N,N-diméthylformamide diméthylacétal est chauffée à une température voisine de 75°C, sous agitation, pendant 2 heures. Le mélange réactionnel est concentré à sec sous pression réduite (20 kPa) à 50°C. L'extrait sec est repris par 50 cm3 d'éther isopropylique, essoré, lavé par 2 fois 10 cm3 d'éther isopropylique. On obtient 16,57 g de (dichloro-2,7 fluoro-6 quinoléinecarbonyle-3)-2 diméthylamino-3 acrylate d'éthyle sous forme d'un solide orangé fondant à 122°C. Ce produit est utilisé sans autre purification pour les étapes ultérieures.

Le (dichloro-2,7 fluoro-6 quinolyl-3)-3 oxo-3 propionate d'éthyle est préparé de la manière suivante :

Une suspension de 38,75 g d'acide dichloro-2,7 fluoro-6 quinoléine carboxylique-3 dans 410 cm3 de trichlorométhane et 24 cm3 de chlorure de thionyle est chauffée à une température voisine de 60°C, sous agitation, pendant 6 heures. La solution obtenue est concentrée à sec sous pression réduite (20 kPa) à 50°C. L'extrait sec est repris 2 fois par au total 200 cm3 de toluène et concentré de nouveau sous pression réduite dans les mêmes conditions que précédemment. Le solide jaune obtenu fondant à 124°C est dissous dans 230 cm3 de tétrahydrofuranne anhydre. La solution obtenue est introduite, goutte à goutte, sous agitation, en 30 minutes, entre 5 et 10°C, dans 200 cm3 d'une solution de chélate de magnésium dans le tétrahydrofuranne dont la préparation est décrite ci-après. On laisse la température remonter à 20°C et agite 1 heure et demie à cette température. La solution obtenue est introduite, goutte à goutte, sous forte agitation à une température voisine de 5°C, dans 1 litre d'acide sulfurique 0,5N. On laisse la température de la suspension obtenue remonter à 20°C et agite encore 2 heures à cette température. On extrait par 1 litre d'acétate d'éthyle, filtre les phases organique et aqueuse sur silice diatomée pour filtration ce qui permet d'éliminer un léger insoluble, extrait la phase aqueuse encore 2 fois par 500 cm3 d'acétate d'éthyle. Les extraits organiques réunis sont lavés par 2 fois 500 cm3 d'eau, séchés sur sulfate de magnésium, filtrés et concentrés à sec sous pression réduite (20 kPa) à 40°C. Le résidu est repris par 100 cm3 d'éther isopropylique à 20°C, essoré et lavé par 2 fois 30 cm3 d'éther isopropylique. On obtient 40,55 g de (dichloro-2,7 fluoro-6 quinolyl-3)-3 oxo-3 propionate d'éthyle sous forme d'un solide beige fondant à 112-114°C. Ce produit est utilisé sans autre purification pour les étapes ultérieures.

Préparation du chélate de magnésium du monomalonate d'éthyle :

A 6,9 g de magnésium en tournure on ajoute progressivement 5 cm3 d'éthanol absolu, 0,2 cm3 de tétrachlorométhane et 2 g de monomalonate d'éthyle. Après échauffement, on ajoute, en 15 minutes, une solution de 23,8 g de monomalonate d'éthyle dans 450 cm3 d'éthanol. Le mélange est chauffé 20 heures à une température voisine de 78°C, concentré sous pression réduite (20 kPa) à 50°C. Le résidu est repris par 2 fois 100 cm3 de toluène et concentré sous pression réduite dans les mêmes conditions que précédemment. La poudre grise obtenue est mise en solution par addition de tétrahydrofuranne anhydre de façon à obtenir un volume total de 200 cm3.

Le monomalonate d'éthyle a été préparé selon la méthode décrite par D.S. Breslow, E. Baumgarten, C.R. Hauser., J. Am. Chem. Soc., 66, 1287 (1944) et distillé sous pression réduite (point d'ébullition=132°C/2,7 kPa).

L'acide dichloro-2,7 fluoro-6 quinoléine carboxylique-3 est préparé de la manière suivante :

A une suspension, sous agitation, refroidie à 10°C, de 69,5 g de dichloro-2,7 fluoro-6 formyl-3 dihydro-1,4 quinoléine dans 282 cm3 de potasse aqueuse 2N et 282 cm3 d'eau, on ajoute en 1 heure en maintenant la température entre 10 et 14°C, une solution de 89,3 g de permanganate de potassium dans 1,4 litre d'eau. On laisse la température remonter à environ 20°C et agite encore 30 minutes à cette température. On ajoute 26 g de dithionite de sodium, agite 10 minutes à température voisine de 20°C, filtre sur silice diatomée pour filtration, lave par 2 fois 250 cm3 d'eau. Le filtrat et les phases aqueuses de lavage sont réunis et additionnés de 90 cm3 d'acide chlorhydrique en solution aqueuse à 35 %. Le précipité formé est extrait par 4 fois 500 cm3 d'acétate d'éthyle. Les extraits organiques réunis sont lavés par 3 fois 500 cm3 d'eau, séchés sur sulfate de magnésium, filtrés et concentrés sous pression réduite (20 kPa) à 50°C. Le résidu est repris par 350 cm3 d'éther éthylique, essoré, lavé par 2 fois 200 cm3 d'éther éthylique. On obtient 45 g d'acide dichloro-2,7 fluoro-6 quinoléine carboxylique-3 sous forme d'un solide beige fondant à 230°C qui est utilisé sans autre purification pour les étapes ultérieures.

La dichloro-2,7 fluoro-6 formyl-3 dihydro-1,4 quinoléine a été préparée de la manière suivante :

A un mélange de 250 cm3 de trichlorométhane et de 54 cm3 de diméthylformamide, on ajoute en 30 minutes, sous agitation, entre 10 et 15°C, 55,6 cm3 de chlorure de phosphoryle et agite 1 heure à température voisine de 20°C. A la solution obtenue, on ajoute en 10 minutes, à environ 20°C, progressivement, sous forte agitation, 52 g de chloro-7 fluoro-6 dihydro-3,4 carbostyrile. La suspension obtenue est chauffée à une température voisine de 60°C et maintenue encore 2 heures, sous agitation à cette température. Le mélange réactionnel est concentré sous pression réduite (20 kPa) à 50°C jusqu'à obtention d'un mélange pâteux. Sous forte agitation, on ajoute un mélange de 250 cm3 d'eau et 250 g de glace pilée. Le solide obtenu est essoré à environ 5°C et lavé par 4 fois 125 cm3 d'eau à 5°C. Le produit humide obtenu et 58 g d'acétate de sodium sont ajoutés simultanément, en 1 heure, à 500 cm3 d'eau à 90°C de manière à maintenir le pH à environ 6. On agite encore 15 minutes à 90°C, laisse la température redescendre à environ 50°C, essore à cette température et lave par 3 fois 250 cm3 d'eau à environ 20°C. On obtient 54,3 g de dichloro-2,7 fluoro-6 formyl-3 dihydro-1,4 quinoléine sous forme d'un solide jaune fondant à 260°C qui est utilisé tel quel pour les étapes ultérieures.

Le chloro-7 fluoro-6 dihydro-3,4 carbostyrile est préparé de la manière suivante :

A 174,4 g de chloro-3' fluoro-4' N-chloro-3 propionanilide, on ajoute sous forte agitation en 5 minutes, 350 g de chlorure d'aluminium. Le mélange solide est chauffé en 30 minutes à environ 60°C. La température monte d'elle-même à environ 80°C et le mélange réactionnel devient liquide. On chauffe alors à 110°C en 15 minutes et maintient entre 110 et 120°C pendant 3 heures. Le mélange réactionnel (à environ 110°C) est versé, en 10 minutes, sous forte agitation, dans un mélange de 550 cm3 d'acide chlorhydrique à 35 % et de 500 g de glace pilée. On laisse la température remonter à 20°C, essore, lave par 6 fois 500 cm3 d'eau.

Le produit humide est recristallisé dans 1,2 litre d'éthanol. On obtient 108 g de chloro-7 fluoro-6 dihydro-3,4 carbostyrile sous forme d'un solide beige fondant à 215°C.

La chloro-3' fluoro-4' N-chloro-3 propionanilide a été préparée de la manière suivante :

A une solution, à température voisine de 55°C, de 291 g de chloro-3 fluoro-4 aniline dans 500 cm3 d'acétone, on ajoute, sous agitation, en 35 minutes, une solution de 127 g de chlorure de l'acide chloro-3 propionique dans 200 cm3 d'acétone et maintient à cette température pendant 2 heures. Après refroidissement à environ 20°C, l'insoluble est éliminé par filtration et lavé par 2 fois 200 cm3 d'acétone. Le filtrat et les lavages réunis sont versés sous agitation dans 2 litres d'eau et 1 kg de glace. On laisse la température remonter à environ 20°C, extrait par 4 fois 500 cm3 de dichlorométhane. Les extraits organiques réunis sont lavés par 3 fois 500 cm3 d'eau, séchés sur sulfate de magnésium, agités 15 minutes avec 6 g de charbon végétal Norit, filtrés sur silice diatomée pour filtration et concentrés sous pression réduite (2,7 kPa) à 50°C. Le solide obtenu est recristallisé dans un mélange de 133 cm3 de cyclohexane et de 67 cm3 d'éther isopropylique. On obtient 176 g de chloro-3' fluoro-4' N-chloro-3 propionanilide sous forme d'un solide beige fondant à 94°C qui est utilisé tel quel pour les étapes ultérieures.

### EXEMPLE 2

L'acide fluoro-7 méthyl-1 oxo-4 (pipérazinyl-1)-8 dihydro-1,4 benzo[b]naphtyridine-1,8 carboxylique-3 est préparé dans les conditions de l'exemple 1 mais à partir de 10 g d'acide chloro-8 fluoro-7 méthyl-1 oxo-4 dihydro-1,4 benzo[b]naphtyridine-1,8 carboxylique-3 et de 28 g de pipérazine, dans 100 cm3 de pyridine. On obtient 5,5 g d'hémihydrate de l'acide fluoro-7 méthyl-1 oxo-4 (pipérazinyl-1)-8 dihydro-1,4 benzo[b]naphtyridine-1,8 carboxylique-3 sous forme d'un solide jaune fondant à 370-375°C.

### EXEMPLE 3

L'acide fluoro-7 méthyl-1 (méthyl-4 pipérazinyl-1)-8 oxo-4 dihydro-1,4 benzo[b]naphtyridine-1,8 carboxylique-3 est préparé dans des conditions analogues à l'exemple 1 mais à partir de 5 g d'acide chloro-8 fluoro-7 méthyl-1 oxo-4 dihydro-1,4 benzo[b]naphtyridine-1,8 carboxylique-3 et de 16 g de méthyl-1 pipérazine dans 50 cm3 de pyridine. Après concentration du mélange réactionnel sous pression réduite le résidu mis en suspension dans 100 cm3 d'eau est additionné de 25 cm3 d'acide acétique. On élimine un très léger insoluble par filtration sur silice diatomée pour filtration. On ajoute au filtrat 200 cm3 de potasse aqueuse 3N, élimine de nouveau un très léger insoluble par filtration sur silice diatomée pour filtration. On ajoute au filtrat 5 cm3 d'acide acétique. Le précipité formé est essoré, lavé par 3 fois 50 cm3 d'eau. Après 2 recristallisations dans 17 cm3 de diméthylformamide à chaque fois, on obtient 3,2 g d'acide fluoro-7 méthyl-1 (méthyl-4 pipérazinyl-1)-8 oxo-4 dihydro-1,4 benzo[b]naphtyridine-1,8 carboxylique-3 sous forme d'un solide jaune fondant à 356°C.

### EXEMPLE 4

L'acide (éthyl-4 pipérazinyl-1)-8 fluoro-7 méthyl-1 oxo-4 dihydro-1,4 benzo[b]naphtyridine-1,8 carboxylique-3 est préparé dans les conditions décrites ci-après à l'exemple 5, mais à partir de 1,85 g d'acide chloro-8 fluoro-7 méthyl-1 oxo-4 dihydro-1,4 benzo[b]naphtyridine-1,8 carboxylique-3 et de 2,75 g d'éthyl-1 pipérazine, dans 20 cm3 de pyridine. On obtient 1,3 g d'acide (éthyl-4 pipérazinyl-1)-8 fluoro-7 méthyl-1 oxo-4 dihydro-1,4 benzo[b]naphtyridine-1,8 carboxylique-3 sous forme d'un solide jaune fondant à 285-286°C.

### EXEMPLE 5

L'acide fluoro-7 [(hydroxy-2 éthyl)-4 pipérazinyl-1]-8 méthyl-1 oxo-4 dihydro-1,4 benzo[b]naphtyridine-1,8 carboxylique-3 est préparé dans les conditions de l'exemple 1 mais à partir de 1,6 g d'acide chloro-8 fluoro-7 méthyl-1 oxo-4 dihydro-1,4 benzo[b]naphtyridine-1,8 carboxylique-3 et de 6,8 g d'(hydroxy-2 éthyl)-1 pipérazine dans 16 cm3 de pyridine. Après concentration à sec du mélange réactionnel sous pression réduite, le résidu est repris par 50 cm3 d'eau. Le mélange est amené à pH 6,9 par addition de 0,4 cm3 d'acide acétique. Le précipité obtenu est essoré, lavé par 2 fois 10 cm3 d'eau, recristallisé 2 fois dans 10 cm3 de diméthylformamide. On obtient 1,1 g d'acide fluoro-7 [(hydroxy-2 éthyl)-4 pipérazinyl-1]-8 méthyl-1 oxo-4 dihydro-1,4 benzo[b]naphtyridine-1,8 carboxylique-3 sous forme d'un solide jaune fondant à 275-276°C.

### EXEMPLE 6

L'acide (diméthyl-3,5 pipérazinyl-1)-8 fluoro-7 méthyl-1 oxo-4 dihydro-1,4 benzo[b]naphtyridine-1,8 carboxylique-3 est préparé dans les conditions de l'exemple 3 mais à partir de 1,7 g d'acide chloro-8 fluoro-7 méthyl-1 oxo-4 dihydro-1,4 benzo[b]naphtyridine-1,8 carboxylique-3 et de 2,5 g de diméthyl-2,6 pipérazine, dans 20 cm3 de pyridine. On obtient 1,1 g d'hémihydrate de l'acide (diméthyl-3,5 pipérazinyl-1)-8 fluoro-7 méthyl-1 oxo-4 dihydro-1,4 benzo[b]naphtyridine-1,8 carboxylique-3 sous forme d'un solide jaune fondant à 294-295°C.

### EXEMPLE 7

L'acide éthyl-1 fluoro-7 (pipérazinyl-1)-8 oxo-4 dihydro-1,4 benzo[b]naphtyridine-1,8 carboxylique-3 est préparé dans les conditions de l'exemple 5 mais à partir de 1,6 g d'acide chloro-8 éthyl-1 fluoro-7 oxo-4 dihydro-1,4 benzo[b]naphtyridine-1,8 carboxylique-3 et de 4,3 g de pipérazine dans 20 cm3 de pyridine. Après 3 recristallisations dans, au total, 300 cm3 de diméthylformamide, on obtient 0,94 g de trihydrate de l'acide éthyl-1 fluoro-7 (pipérazinyl-1)-8 oxo-4 dihydro-1,4 benzo[b]naphtyridine-1,8 carboxylique-3 sous forme d'un solide jaune fondant à 320-322°C.

L'acide chloro-8 éthyl-1 fluoro-7 oxo-4 dihydro-1,4 benzo[b]naphtyridine-1,8 carboxylique-3 est préparé dans les conditions de l'exemple 1, mais à partir de 10,5 g de chloro-8 fluoro-7 éthoxycarbonyl-3 éthyl-1 oxo-4 dihydro-1,4 benzo[b]naphtyridine-1,8. On obtient 9,3 g d'acide chloro-8 éthyl-1 fluoro-7 oxo-4 dihydro-1,4 benzo[b]naphtyridine-1,8 carboxylique-3 sous forme d'un solide beige fondant à 380°C qui est utilisé sans autre purification pour les étapes ultérieures.

La chloro-8 éthoxycarbonyl-3 éthyl-1 fluoro-7 oxo-4 dihydro-1,4 benzo[b]naphtyridine-1,8 est préparée de la manière suivante :

Dans une suspension sous agitation de 13,5 g de (dichloro-2,7 fluoro-6 quinoléinecarbonyle-3)-2 diméthylamino-3 acrylate d'éthyle dans 135 cm3 d'éthanol, on ajoute, en 5 minutes, entre 10 et 15°C, 16 g d'éthylamine, laisse la température remonter à environ 20°C, ajoute 0,5 g de DBU et chauffe sous agitation, pendant 2 heures, à une température voisine de 75°C. Après refroidissement à une température voisine de 20°C, le précipité est essoré, lavé par 2 fois 100 cm3 d'éthanol, 2 fois 100 cm3 d'éther éthylique. On obtient 10,4 g de chloro-8 éthoxycarbonyl-3 éthyl-1 fluoro-7 oxo-4 dihydro-1,4 benzo[b]naphtyridine-1,8 sous forme d'un solide jaune fondant à 300-301°C, qui est utilisé sans autre purification pour les étapes ultérieures.

### EXEMPLE 8

L'acide éthyl-1 fluoro-7 (méthyl-4 pipérazinyl-1)-8 oxo-4 dihydro-1,4 benzo[b]naphtyridine-1,8 carboxylique-3 est préparé dans les conditions de l'exemple 5 mais à partir de 1,6 g d'acide chloro-8 éthyl-1 fluoro-7 oxo-4 dihydro-1,4 benzo[b]naphtyridine-1,8 carboxylique-3 et de 4,5 g de méthyl-4 pipérazine dans 16 cm3 de pyridine. Après 4 recristallisations dans, au total, 120 cm3 de diméthylformamide, on obtient 1,2 g d'acide éthyl-1 fluoro-7 (méthyl-4 pipérazinyl-1)-8 oxo-4 dihydro-1,4 benzo[b]naphtyridine-1,8 carboxylique-3 sous forme d'un solide jaune fondant à 285-286°C solvaté par 1 % d'eau.

### EXEMPLE 9

L'acide éthyl-1 fluoro-7 (méthyl-3 pipérazinyl-1)-8 oxo-4 dihydro-1,4 benzo[b]naphtyridine-1,8 carboxylique-3 est préparé dans les conditions de l'exemple 1 mais à partir de 2,1 g d'acide chloro-8 éthyl-1 fluoro-7 oxo-4 dihydro-1,4 benzo[b]naphtyridine-1,8 carboxylique-3, de 20 cm3 de pyridine, et de 2,4 g de méthyl-2 pipérazine. Après reprise par l'éthanol et concentration à sec sous pression réduite (20 kPa à 50°C), le résidu solide est repris par 20 cm3 d'eau et 10 cm3 de potasse 2N. La solution aqueuse obtenue est lavée par 2 fois 20 cm3 de trichlorométhane, additionnée de 10 cm3 d'acide acétique, lavée de nouveau par 2 fois 40 cm3 de trichlorométhane. On ajoute 23 cm3 de potasse 4,5 N, chauffe la suspension obtenue à une température voisine de 90°C. Après refroidissement à une température voisine de 20°C, le précipité est essoré, lavé par 3 fois 10 cm3 d'eau, 2 fois 10 cm3 d'éthanol. Après 2 recristallisations dans 120 cm3 de diméthylformamide à chaque fois, on obtient 1,7 g d'acide éthyl-1 fluoro-7 (méthyl-3 pipérazinyl-1)-8 oxo-4 dihydro-1,4 benzo[b]naphtyridine-1,8 carboxylique-3 sous forme d'un solide jaune fondant à 310-312°C.

### EXEMPLE 10

L'acide éthyl-1 (éthyl-4 pipérazinyl-1)-8 fluoro-7 oxo-4 dihydro-1,4 benzo[b]naphtyridine-1,8 carboxylique-3 est préparé dans les conditions de l'exemple 5, mais à partir de 1,6 g d'acide chloro-8 éthyl-1 fluoro-7 oxo-4 dihydro-1,4 benzo[b]naphtyridine-1,8 carboxylique-3 et de 2,3 g d'éthyl-1 pipérazine dans 16 cm3 de pyridine. On obtient 1,4 g d'acide éthyl-1 (éthyl-4 pipérazinyl-1)-8 fluoro-7 oxo-4 dihydro-1,4 benzo[b]naphtyridine-1,8 carboxylique-3 sous forme d'un solide jaune fondant à 287-288°C, solvaté par 1,6 % d'eau.

### EXEMPLE 11

L'acide éthyl-1 fluoro-7 [(hydroxy-2 éthyl)-4 pipérazinyl-1]-8 oxo-4 dihydro-1,4 benzo[b]naphtyridine-1,8 carboxylique-3 est préparé dans les conditions de l'exemple 5, mais à partir de 1,6 g de l'acide chloro-8 éthyl-1 fluoro-7 oxo-4 dihydro-1,4 benzo[b]naphtyridine-1,8 carboxylique-3 et de 2,6 g de (hydroxy-2 éthyl)-1 pipérazine dans 16 cm3 de pyridine. On obtient 1,3 g d'acide éthyl-1 fluoro-7 [(hydroxy-2 éthyl)-4 pipérazinyl-1]-8 oxo-4 dihydro-1,4 benzo[b]naphtyridine-1,8 carboxylique-3 sous forme d'un solide jaune fondant à 264-265°C.

### EXEMPLE 12

L'acide fluoro-7 méthylamino-1 oxo-4 (pipérazinyl-1)-8 dihydro-1,4 benzo[b]naphtyridine-1,8 carboxylique-3 est préparé dans les conditions de l'exemple 5 mais à partir de 2,25 g d'acide chloro-8 fluoro-7 méthylamino-1 oxo-4 dihydro-1,4 benzo[b]naphtyridine-1,8 carboxylique-3 et de 2,4 g de pipérazine dans 30 cm3 de pyridine. Après 3 recristallisations dans, au total, 400 cm3 de diméthylformamide, on obtient 0,82 g d'acide fluoro-7 méthylamino-1 oxo-4 (pipérazinyl-1)-8 dihydro-1,4 benzo[b]naphtyridine-1,8 carboxylique-3 sous forme d'un solide jaune foncé fondant à 322-324°C solvaté par 13,6 % de diméthylformamide.

L'acide chloro-8 fluoro-7 méthylamino-1 oxo-4 dihydro-1,4 benzo[b]naphtyridine-1,8 carboxylique-3 peut être préparé dans les conditions suivantes :

Une suspension de 16,4 g de chloro-8 éthoxycarbonyl-3 fluoro-7 N-formyl N-méthyl amino-1 oxo-4 dihydro-1,4 benzo[b]naphtyridine-1,8 dans 164 cm3 d'acide acétique et 164 cm3 d'acide chlorhydrique en solution aqueuse à 17,5 % est chauffée à une température voisine de 100°C, sous agitation pendant 4 heures. Après refroidissement à température voisine de 10°C, on ajoute entre 10 et 20°C, 165 cm3 de lessive de soude à 30 %. Le produit est essoré, lavé par 3 fois 150 cm3 d'eau, 3 fois 150 cm3 d'éthanol et 3 fois 150 cm3 d'éther éthylique. On obtient 13,64 g d'acide chloro-8 fluoro-7 méthylamino-1 oxo-4 dihydro-1,4 benzo[b]naphtyridine-1,8 carboxylique-3 sous forme d'un solide jaune fondant à 354-356°C, qui est utilisé sans autre purification pour les étapes ultérieures.

La chloro-8 éthoxycarbonyl-3 fluoro-7 (N-formyl N-méthyl amino)-1 oxo-4 dihydro-1,4 benzo[b]naphtyridine-1,8 est préparée dans les conditions de l'exemple 1 mais à partir de 19,25 g de (dichloro-2,7 fluoro-6 quinoléinecarbonyle-3)-2 diméthylamino-3 acrylate d'éthyle, de 4,05 g de N-formyl N-méthyl hydrazine et de 1,6 g de DBU, dans 200 cm3 d'éthanol. On obtient 16,4 g de chloro-8 éthoxycarbonyl-3 fluoro-7 (N-formyl N-méthyl amino)-1 oxo-4 dihydro-1,4 benzo[b]naphtyridine-1,8 sous forme de solide incolore fondant à 296-298°C, qui est utilisé sans autre purification pour les étapes ultérieures.

La N-formyl-N-méthylhydrazine peut être préparée selon la méthode décrite par Carl Th. Pedersen, Acta Chem. Scand., 18(9), 2199 (1964).

### EXEMPLE 13

L'acide fluoro-7 méthylamino-1 (méthyl-4 pipérazinyl-1)-8 oxo-4 dihydro-1,4 benzo[b]naphtyridine-1,8 carboxylique-3 est préparé dans des conditions analogues à l'exemple 1 mais à partir de 1,93 g d'acide chloro-8 fluoro-7 méthylamino-1 oxo-4 dihydro-1,4 benzo[b]naphtyridine-1,8 carboxylique-3, de 2,4 g de méthyl-1 pipérazine, de 20 cm3 de pyridine. Après 2 recristallisations dans 15 cm3 de diméthylformamide à chaque fois, on obtient 0,9 g d'acide fluoro-7 méthylamino-1 (méthyl-4 pipérazinyl-1)-8 oxo-4 dihydro-1,4 benzo[b]naphtyridine-1,8 carboxylique-3 sous forme d'un solide jaune fondant à 263-264°C.

### EXEMPLE 14

L'acide fluoro-7 méthylamino-1 (méthyl-3 pipérazinyl-1)-8 oxo-4 dihydro-1,4 benzo[b]naphtyridine-1,8 carboxylique-3 est préparé dans les conditions de l'exemple 5 mais à partir de 3,2 g d'acide chloro-8 fluoro-7 méthylamino-1 oxo-4 dihydro-1,4 benzo[b]naphtyridine-1,8 carboxylique-3 et de 4 g de méthyl-2 pipérazine dans 40 cm3 de pyridine. Le produit brut obtenu est repris par 30 cm3 d'eau et 7 cm3 de potasse aqueuse 2N. On élimine un très léger insoluble par filtration sur silice diatomée. Le filtrat est lavé par 2 fois 20 cm3 d'éther éthylique puis précipité par addition de 3,5 cm3 d'acide méthanesulfonique 4N. Le précipité obtenu est essoré, lavé par 3 fois 20 cm3 d'eau,3 fois 20 cm3 d'éthanol. On obtient 2,2 g d'acide fluoro-7 méthylamino-1 (méthyl-3 pipérazinyl-1)-8 oxo-4 dihydro-1,4 benzo[b]naphtyridine-1,8 carboxylique-3 sous forme d'un solide jaune foncé fondant à 343-345°C, solvaté par 3,7 % d'eau.

### EXEMPLE 15

L'acide cyclopropyl-1 fluoro-7 oxo-4 (pipérazinyl-1)-8 dihydro-1,4 benzo[b]naphtyridine-1,8 carboxylique-3 est préparé dans les conditions de l'exemple 5, mais à partir de 1 g d'acide chloro-8 cyclopropyl-1 fluoro-7 oxo-4 dihydro-1,4 benzo[b]naphtyridine-1,8 carboxylique-3 et de 2,6 g de pipérazine, dans 10 cm3 de pyridine. On obtient 0,6 g de dihydrate de l'acide cyclopropyl-1 fluoro-7 oxo-4 (pipérazinyl-1)-8 dihydro-1,4 benzo[b]naphtyridine-1,8 carboxylique-3 sous forme d'un solide jaune fondant à 342-343°C.

L'acide chloro-8 cyclopropyl-1 fluoro-7 oxo-4 dihydro-1,4 benzo[b]naphtyridine-1,8 carboxylique-3 est préparé dans les conditions de l'exemple 1, mais à partir de 6,1 g de chloro-8 cyclopropyl-1 éthoxycarbonyl-3 fluoro-7 oxo-4 dihydro-1,4 benzo[b]naphtyridine-1,8. On obtient 4,85 g d'acide chloro-8 cyclopropyl-1 fluoro-7 oxo-4 dihydro-1,4 benzo[b]naphtyridine-1,8 carboxylique-3 sous forme d'un solide jaune fondant à 330°C qui est utilisé sans autre purification pour les étapes ultérieures.

La chloro-8 cyclopropyl-1 éthoxycarbonyl-3 fluoro-7 oxo-4 dihydro-1,4 benzo[b]naphtyridine-1,8 est préparée dans les conditions suivantes :

Une solution de 20,6 g de (dichloro-2,7 fluoro-6 quinoléinecarbonyle-3)-2 diméthylamino-3 acrylate d'éthyle et de 6 g de cyclopropylamine dans 100 cm3 de trichlorométhane est agitée à une température voisine de 20°C pendant 24 heures. Le mélange réactionnel est concentré sous pression réduite (20 kPa) à 50°C. Le résidu est repris par 180 cm3 d'éthanol et 10 g de DBU et la solution obtenue chauffée à une température voisine de 78°C pendant 4 heures. Après refroidissement à une température voisine de 20°C, le précipité obtenu est essoré et lavé par 2 fois 60 cm3 d'éthanol. On obtient 13,65 g de chloro-8 cyclopropyl-1 éthoxycarbonyl-3 fluoro-7 oxo-4 dihydro-1,4 benzo[b]naphtyridine-1,8 sous forme d'un solide jaune pâle fondant à 256°C qui est utilisé sans autre purification pour les étapes ultérieures.

### EXEMPLE 16

L'acide cyclopropyl-1 fluoro-7 (méthyl-4 pipérazinyl-1)-8 oxo-4 dihydro-1,4 benzo[b]naphtyridine-1,8 carboxylique-3 est préparé dans les conditions de l'exemple 5 mais à partir de 1 g d'acide chloro-8 cyclopropyl-1 fluoro-7 oxo-4 dihydro-1,4 benzo[b]naphtyridine-1,8 carboxylique-3, de 3 g de méthyl-1 pipérazine dans 10 cm3 de pyridine. Après une recristallisation dans 10 cm3 de diméthylformamide, on obtient 0,63 g d'acide cyclopropyl-1 fluoro-7 (méthyl-4 pipérazinyl-1)-8 oxo-4 dihydro-1,4 benzo[b]naphtyridine-1,8 carboxylique-3 sous forme d'un solide jaune fondant à 250°C.

### EXEMPLE 17

L'acide cyclopropyl-1 fluoro-7 (méthyl-3 pipérazinyl-1)-8 oxo-4 dihydro-1,4 benzo[b]naphtyridine-1,8 carboxylique-3 est préparé dans les conditions de l'exemple 1 mais à partir de 1 g d'acide chloro-8 cyclopropyl-1 fluoro-7 oxo-4 dihydro-1,4 benzo[b]naphtyridine-1,8 carboxylique-3 et de 3 g de méthyl-2 pipérazine, dans 10 cm3 de pyridine. Le produit pur est obtenu après une purification supplémentaire par recristallisation dans 200 cm3 de diméthylformamide On obtient 0,5 g d'hémihydrate de l'acide cyclopropyl-1 fluoro-7 (méthyl-3 pipérazinyl-1)-8 oxo-4 dihydro-1,4 benzo[b]naphtyridine-1,8 carboxylique-3 sous forme d'un solide jaune fondant à 343°C.

### EXEMPLE 18

L'acide cyclopropyl-1 (éthyl-4 pipérazinyl-1)-8 fluoro-7 oxo-4 dihydro-1,4 benzo[b]naphtyridine-1,8 carboxylique-3 est préparé dans les conditions de l'exemple 5, mais à partir de 2 g d'acide chloro-8 cyclopropyl-1 fluoro-7 oxo-4 dihydro-1,4 benzo[b]naphtyridine-1,8 carboxylique-3 et de 2,74 g d'éthyl-1 pipérazine dans 20 cm3 de pyridine. Le produit pur est isolé après une première recristallisation dans 105 cm3 d'éthanol à 25 % de diméthylformamide suivie d'une seconde recristallisation dans 75 cm3 d'éthanol à 50 % de diméthylformamide. On obtient 0,67 g d'acide cyclopropyl-1 (éthyl-4 pipérazinyl-1)-8 fluoro-7 oxo-4 dihydro-1,4 benzo[b]naphtyridine-1,8 carboxylique-3 sous forme d'un solide jaune vert fondant à 254°C.

### EXEMPLE 19

L'acide cyclopropyl-1 fluoro-7 [(hydroxy-2 éthyl)-4 pipérazinyl-1]-8 oxo-4 dihydro-1,4 benzo[b]naphtyridine-1,8 carboxylique-3 est préparé dans des conditions analogues à l'exemple 5, mais à partir de 4 g d'acide chloro-8 cyclopropyl-1 fluoro-7 oxo-4 dihydro-1,4 benzo[b]naphtyridine-1,8 carboxylique-3 et de 6,2 g d'(hydroxy-2 ethyl)-1 pipérazine dans 40 cm3 de pyridine. Le mélange réactionnel est chauffé pendant 22 heures à une température voisine de 115°C. Le produit pur est isolé après 3 recristallisations dans 3 fois 200 cm3 d'éthanol à 10% de diméthylformamide à chaque fois. On obtient 0,94 g d'acide cyclopropyl-1 fluoro-7 [(hydroxy-2 éthyl)-4 pipérazinyl-1]-8 oxo-4 dihydro-1,4 benzo[b]naphtyridine-1,8 carboxylique-3 sous forme d'un solide jaune fondant à 255°C.

### EXEMPLE 20

L'acide fluoro-7 oxo-4 (pipérazinyl-1)-8 tertiobutyl-1 dihydro-1,4 benzo[b]naphtyridine-1,8 carboxylique-3 est préparé dans les conditions de l'exemple 5, mais à partir de 1,7 g d'acide chloro-8 fluoro-7 oxo-4 tertiobutyl-1 dihydro-1,4 benzo[b]naphtyridine-1,8 carboxylique-3 et de 4,3 g de pipérazine, dans 20 cm3 de pyridine. Le produit pur est obtenu après une seule recristallisation dans 20 cm3 de diméthylformamide. On isole 1,25 g d'acide fluoro-7 oxo-4 (pipérazinyl-1)-8 tertiobutyl-1 dihydro-1,4 benzo[b]naphtyridine-1,8 carboxylique-3 sous forme d'un solide jaune fondant à 290°C, solvaté par 4,5 % d'eau.

L'acide chloro-8 fluoro-7 oxo-4 tertiobutyl-1 dihydro-1,4 benzo[b]naphtyridine-1,8 carboxylique-3 peut être préparé de la manière suivante :

Une suspension de 1,88 g de chloro-8 éthoxycarbonyl-3 fluoro-7 oxo-4 tertiobutyl-1 dihydro-1,4 benzo[b]naphtyridine-1,8 dans 10 cm3 d'éthanol, 5 cm3 d'eau et 15 cm3 de potasse aqueuse 2N est chauffée à une température voisine de 75°C, sous agitation pendant une heure. La solution obtenue est additionnée de 2 cm3 d'acide acétique. Le précipité formé est essoré, lavé par 3 fois 10 cm3 d'eau, 3 fois 10 cm3 d'éthanol. Après une recristallisation dans 50 cm3 de diméthylformamide, on obtient 1,7 g d'acide chloro-8 fluoro-7 oxo-4 tertiobutyl-1 dihydro-1,4 benzo[b]naphtyridine-1,8 carboxylique-3 sous forme d'un solide jaune fondant à 398°C.

La chloro-8 éthoxycarbonyl-3 fluoro-7 oxo-4 tertiobutyl-1 dihydro-1,4 benzo[b]naphtyridine-1,8 est préparée dans les conditions de l'exemple 17, mais à partir de 8,86 g (dichloro-2,7 fluoro-6 quinoléinecarbonyle-3)-2 diméthylamino-3 acrylate d'éthyle, de 4,03 g de tertiobutylamine dans 45 cm3 de trichlorométhane, puis dans 4,53 g de DBU et 45 cm3 d'éthanol. On obtient 5 g de chloro-8 éthoxycarbonyl-3 fluoro-7 oxo-4 tertiobutyl-1 dihydro-1,4 benzo[b]naphtyridine-1,8 sous forme d'un solide jaune fondant à 239°C.

### EXEMPLE 21

Une suspension de 2 g d'acide chloro-8 fluoro-7 méthyl-1 oxo-4 dihydro-1,4 benzo [b] naphtyridine-1,8 carboxylique-3 dans 7,44 g de diméthyl-2,2 pipérazine et 20 cm3 de pyridine est chauffée à une température voisine de 115°C pendant 44 heures. Le mélange réactionnel est concentré à sec sous pression réduite (20 kPa) à environ 60°C. Le résidu est repris par 50 cm3 d'éthanol et concentré de nouveau, sous pression réduite, dans les conditions précédentes. le solide obtenu est repris par 50 cm3 d'éther éthylique, essoré, lavé par 2 fois 30 cm3 du même solvant et repris par 120 cm3 d'eau et 2 g d'acide méthanesulfonique. Un très faible insoluble est éliminé par filtration sur silice diatomée. La solution obtenue est additionnée de 2 cm3 de potasse aqueuse à 50 %. Le précipité formé est essoré, lavé par 3 fois 25 cm3 d'eau et 1 fois 50 cm3 d'éthanol à environ 50°C. On obtient 1,6 g d'acide (diméthyl-3,3 pipérazinyl-1)-8 fluoro-7 méthyl-1 oxo-4 dihydro-1,4 benzo [b] naphtyridine-1,8 carboxylique-3 sous forme d'un solide jaune fondant à 362-365°C, solvaté par 4,9 % d'eau.

### EXEMPLE 22

Une suspension de 1,2 g d'acide chloro-8 fluoro-7 (fluoro-2 éthyl)-1 oxo-4 dihydro-1,4 benzo [b] naphtyridine-1,8 carboxylique-3 dans 12 cm3 de pyridine et 3,52 g de méthyl-1 pipérazine est chauffée, sous agitation, à une température voisine de 110°C pendant 6 heures. Le mélange réactionnel est concentré sous pression réduite (20kPa) à environ 60°C. Le résidu est repris par 15 cm3 d'eau et 2 cm3 d'acide acétique. On élimine un léger insoluble par filtration sur silice diatomée. Le filtrat est additionné de 6 cm3 de potasse aqueuse à 20%. On élimine de nouveau un très léger insoluble par filtration sur silice diatomée. Le filtrat est additionné de 0,6 cm3 d'acide acétique. Le précipité obtenu est essoré, lavé par 2 fois 5 cm3 d'eau, 2 fois 5 cm3 d'éthanol et recristallisé 2 fois dans 10 cm3 de diméthylformamide à chaque fois. On obtient 0,6 g d'acide fluoro-7 (fluoro-2 éthyl)-1 (méthyl-4 pipérazinyl-1)-8 oxo-4 dihydro-1,4 benzo [b] naphtyridine-1,8 carboxylique-3 sous forme d'un solide jaune fondant à 306-308°C.

L'acide chloro-8 fluoro-7 (fluoro-2 éthyl)-1 oxo-4 dihydro-1,4 benzo [b] naphtyridine-1,8 carboxylique-2 a été préparé dans les conditions de l'exemple 26, mais à partir de 2,2 g de chloro-8 éthoxycarbonyl-3 fluoro-7 (fluoro-2 éthyl)-1 oxo-4 dihydro-1,4 benzo [b] naphtyridine-1,8. Après 2 recristallisations dans 10 cm3 de diméthylformamide à chaque fois, on obtient 1,4 g d'acide chloro-8 fluoro-7 (fluoro-2 éthyl)-1 oxo-4 dihydro-1,4 benzo [b] naphtyridine-1,8 carboxylique-3 sous forme d'un solide jaune fondant à 310°C.

La chloro-8 éthoxycarbonyl-3 fluoro-7 (fluoro-2 éthyl)-1 oxo-4 dihydro-1,4 benzo [b] naphtyridine-1,8 est préparé de la manière suivante:

Une suspension de 1,9 g de chlorhydrate de fluoro-2 éthylamine dans 25 cm3 de trichlorométhane est additionnée de 2,7 cm3 de triéthylamine. A la solution obtenue, on ajoute à environ 20°C, 2,5 g de (dichloro-2,7 fluoro-6 quinoléinecarbonyle-3)-2 diméthylamino-3 acrylate d'éthyle. Après 16 heures d'agitation à cette température, la solution est concentrée à sec sous pression réduite (20kPa) à environ 50°C. Le résidu est repris par 20 cm3 d'éthanol et 3 cm3 de triéthylamine et chauffé à environ 75°C, sous agitation pendant 2 heures. Après refroidissement à environ 20°C, l'insoluble est essoré, lavé par 2 fois 10 cm3 d'éthanol, 2 fois 10 cm3 d'éther isopropylique. On obtient 1,9 g de chloro-8 éthoxycarbonyl-3 fluoro-7 (fluoro-2 éthyl)-1 oxo-4 dihydro-1,4 benzo [b] naphtyridine-1,8 sous forme d'un solide jaune fondant à 268°C qui est utilisé sans autre purification pour les étapes ultérieures.

### EXEMPLE 23

Une suspension de 2 g d'acide difluoro-7,8 méthoxy-1 oxo-4 dihydro-1,4 benzo [b] naphtyridine-1,8 carboxylique-3 dans 2,8 g de pipérazine et 40 cm3 de diméthylsulfoxyde est agitée pendant 15 minutes à une température voisine de 40°C. Après refroidissement à environ 20°C, le mélange réactionnel est versé dans 150 cm3 d'eau, additionné de 27,75 cm3 d'acide méthanesulfonique 2N. Un très faible insoluble est éliminé par filtration sur silice diatomée. La solution obtenue est additionnée de 15 cm3 de potasse aqueuse 2N. Le précipité formé est essoré, lavé par 3 fois 15 cm3 d'eau, repris par 100 cm3 de diméthylformamide et chauffé sous agitation pendant 10 minutes à une température voisine de 150°C. La suspension est refroidie à environ 100°C ; l'insoluble est essoré, repris par 100 cm3 d'éthanol, chauffé à une température voisine de 75°C, pendant 1 heure. L'insoluble est essoré à environ 50°C et lavé par 40 cm3 du même solvant, à la même température que précédemment. On obtient 1,8 g d'acide fluoro-7 méthoxy-1 oxo-4 (pipérazinyl-1)-8 dihydro-1,4 benzo [b] naphtyridine-1,8 carboxylique-3 sous forme d'un solide brun fondant à 298-300°C, solvaté par 2,4 % d'eau.

L'acide difluoro-7,8 méthoxy-1 oxo-4 dihydro-1,4 benzo[b] naphtyridine-1,8 carboxylique-3 est préparé dans les conditions suivantes :

Une suspension de 2,78 g d'éthoxycarbonyl-3 difluoro-7,8 méthoxy-1 oxo-4 dihydro-1,4 benzo [b] naphtyridine-1,8 dans 30 cm3 d'acide chlorhydrique à 17,5 % et 30 cm3 d'acide acétique est chauffée à une température voisine de 100°C pendant 1 heure. Après refroidissement à environ 20°C, le mélange réactionnel est versé dans 100 cm3 d'eau. Le précipité formé est essoré, lavé par 3 fois 30 cm3 d'eau et 2 fois 5 cm3 d'éthanol. Après 1 recristallisation dans 100 cm3 de diméthylformamide à 20 % d'éthanol, on obtient 2,03 g d'acide difluoro-7,8 méthoxy-1 oxo-4 dihydro-1,4 benzo[b] naphtyridine-1,8 carboxylique-3 sous forme d'un solide jaune fondant à 325-327°C.

L'éthoxycarbonyl-3 difluoro-7,8 méthoxy-1 oxo-4 dihydro-1,4 benzo [b] naphtyridine-1,8 est préparé dans les conditions suivantes :

Une suspension de 1,7 de chlorhydrate de méthoxylamine dans 40 cm3 de trichlorométhane est additionnée de 2,13 g de triéthylamine. Après 15 minutes d'agitation à une température voisine de 20°C, la solution obtenue est additionnée de 3,69 g de (chloro-2 difluoro-6,7 quinoléinecarbonyle-3)-2 diméthylamino-3 acrylate d'éthyle et agitée pendant 4 heures et demie à environ 20°C. Le mélange réactionnel est concentré à sec sous pression réduite (20 kPa) à une température voisine de 50°C. Le résidu est repris par 70 cm3 d'éthanol, 3,6 g de triéthylamine et chauffé pendant 30 minutes à une température voisine de 75°C. Après refroidissement à environ 20°C, le précipité obtenue est essoré et lavé par 3 fois 30 cm3 d'éthanol. On obtient 2,67 g d'éthoxycarbonyl-3 difluoro-7,8 méthoxy-1 oxo-4 dihydro-1,4 benzo [b] naphtyridine-1,8 sous forme d'un solide jaune pâle fondant à 266-268°C.

Le (chloro-2 difluoro-6,7 quinoléinecarbonyle-3)-2 diméthylamino-3 acrylate d'éthyle est préparé de la manière suivante :

Une suspension de 6,17 g (chloro-2 difluoro-6,7 quinolyl-3)-3 oxo-3 propionate d'éthyle dans 7,15 g de N,N-diméthylformamidediméthylacétal et 60 cm3 d'acétate d'éthyle est chauffée à une température voisine de 75°C pendant 1 heure 15 minutes. Le mélange réactionnel est concentré à sec sous pression réduite (20 kPa) à environ 50°C. Le résidu est repris par 50 cm3 d'éther isopropylique, essoré, lavé par 3 fois 25 cm3 du même solvant. On obtient 6,65 g de (chloro-2 difluoro-6,7 quinoléinecarbonyle-3)-2 diméthylamino-3 acrylate d'éthyle sous forme d'un solide orangé fondant à 140°C.

Le (chloro-2 difluoro-6,7 quinolyl-3)-3 oxo-3 propionate d'éthyle est préparé de la manière suivante :

Une suspension de 14,13 g d'acide chloro-2 difluoro-6,7 quinoléine carboxylique-3 dans 29 cm3 de chlorure de thionyle et 220 cm3 de trichlorométhane est chauffée à une température voisine de 60°C pendant 4 heures. La solution obtenue est concentrée à sec sous pression réduite (20 kPa) à environ 60°C. Le résidu obtenu est repris par 75 cm3 de n-hexane, essoré, lavé par 2 fois 60 cm3 du même solvant. Les 14,4 g de solide jaune obtenu sont mis en solution dans 115 cm3 de tétrahydrofuranne. Cette solution est introduite, goutte à goutte, sous agitation, en 35 minutes, entre 5 et 10°C, dans 70 cm3 d'une solution de chélate de magnésium du monomalonate d'éthyle dans le tétrahydrofuranne préparé dans les conditions décrites ci-après. On laisse la température remonter à environ 20°C, et agite encore 2 heures dans ces conditions. La solution obtenue est introduite, goutte à goutte, sous agitation, en 30 minutes, à une température voisine de 5°C, dans 560 cm3 d'acide sulfurique 0,5 N. On laisse la température de la suspension obtenue remonter à 20°C et agite encore 1 heure et demie à cette température. On extrait par 3 fois 250 cm3 d'acétate d'éthyle. Les extraits organiques réunis sont lavés par 2 fois 250 cm3 d'eau, séchés sur sulfate de magnésium, filtrés, et concentrés à sec, sous pression réduite (20 kPa) à 50°C. Le résidu obtenu est repris par 50 cm3 de n-hexane à 20 % d'éther isopropylique, essoré, lavé par 10 cm3 du même mélange et recristallisé dans 60 cm3 d'isopropanol à 30 % de n-hexane. On obtient 11,84 g de (chloro-2 difluoro-6,7 quinolyl-3)-3 oxo-3 propionate d'éthyle sous forme d'un solide crème fondant à 107°C.

Préparation du chélate de magnésium du monomalonate d'éthyle:

A 2,78 g de magnésium en tournure on ajoute progressivement 2 cm3 d'éthanol absolu, 0,1 cm3 de tétrachlorométhane et 1 g de monomalonate d'éthyle. Après échauffement, on ajoute, en 15 minutes, une solution de 9 g de monomalonate d'éthyle dans 180 cm3 d'éthanol. Le mélange est chauffé 20 heures à une température voisine de 75°C, concentré sous pression réduite (20 kPa) à 50°C. Le résidue est repris par 2 fois 100 cm3 de toluène et concentré sous pression réduite dans les mêmes conditions que précédemment. La poudre grise obtenue est mise en solution par addition de tétrahydrofuranne anhydre de façon à obtenir un volume total de 70 cm3.

L'acide chloro-2 difluoro-6,7 quinoléine carboxylique-3 a été préparé de la manière suivante :

A une suspension, sous agitation, refroidie à 10°C, de 70,18 g de chloro-2 difluoro-6,7 formyl-3 dihydro-1,4 quinoléine dans 970 cm3 de potasse aqueuse N, on ajoute, en 1 heure, en maintenant la température entre 10 et 14°C, une solution de 115 g de permanganate de potassium dans 1,215 litre d'eau. On laisse la température remonter à environ 20°C et agite encore 30 minutes à cette température. On ajoute 38,5 g de dithionite de sodium, agite 10 minutes à une température voisine de 20°C, filtre sur silice diatomée, lave par 3 fois 200 cm3 d'eau. Le filtrat et les phases aqueuses de lavage sont réunis et additionnés de 140 cm3 d'acide chlorhydrique en solution aqueuse à 35 %. Le précipité formé est extrait par 4 fois 800 cm3 d'acétate d'éthyle. Les extraits organiques réunis sont lavés par 2 fois 500 cm3 d'eau, séchés sur sulfate de magnésium, filtrés et concentrés sous pression réduite (20 kPa) à 50°C. Le résidu est repris par 400 cm3 d'éther éthylique, essoré, lavé par 2 fois 200 cm3 du même solvant. On obtient 49,2 g d'acide chloro-2 difluoro-6,7 quinoléine carboxylique-3 sous forme d'un solide beige fondant à 232°C qui est utilisé, sans autre purification, pour les étapes ultérieures.

La chloro-2 difluoro-6,7 formyl-3 dihydro-1,4 quinoléine a été préparée de la manière suivante :

A un mélange de 800 cm3 de trichlorométhane et de 74,35 cm3 de diméthylformamide, on ajoute en 30 minutes, sous agitation, entre 10 et 15°C, 76,9 cm3 de chlorure de phosphoryle et agite 1 heure à température voisine de 20°C. A la solution obtenue, on ajoute en 10 minutes à environ 20°C,sous forte agitation, 65,8 g de difluoro-6,7 dihydro-3,4 carbostyrile. La suspension obtenue est chauffée à une température voisine de 60°C et maintenue à cette température pendant 2 heures. Le mélange réactionnel est concentré sous pression réduite (20 kPa) à 50°C jusqu'à obtention d'un mélange pâteux. Sous forte agitation on ajoute un mélange de 500 g de glace et 500 cm3 d'eau. Le solide obtenu est essoré à environ 5°C et lavé par 3 fois 300 cm3 d'eau à 5°C. Le produit humide obtenu et 60 g d'acétate de sodium sont ajoutés simultanément, en 1 heure, à 1,5 litre d'eau à 90°C, de manière à maintenir le pH à environ 6. On agite encore 30 minutes à 90°C, laisse la température redescendre à environ 50°C, essore à cette température et lave par 3 fois 300 cm3 d'eau à environ 20°C. On obtient 70,18 g de chloro-2 difluoro-6,7 formyl-3 dihydro-1,4 quinoléine sous forme d'un solide jaune fondant à 260°C qui est utilisé tel quel pour les étapes ultérieures.

Le difluoro-6,7 dihydro-3,4 carbostyrile est obtenu de la manière suivante :

A 67 g de difluoro-3',4' N-chloro-3 propionanilide, on ajoute sous forte agitation, 134 g de chlorure d'aluminium, puis après environ 2 minutes, on ajoute, de nouveau, par petites fractions, en 15 minutes, 135,9 g de difluoro-3',4' N-chloro-3 propionanilide et 272 g de chlorure d'aluminium. La température monte d'elle-même à environ 60°C et le mélange réactionnel devient liquide. On chauffe alors à 110°C en 20 minutes et maintient entre 110 et 120°C pendant 2 heures. Le mélange réactionnel (à environ 110°C) est versé, en 10 minutes, sous forte agitation dans un mélange de 840 cm3 d'acide chlorhydrique à 35 % et de 1 kg de glace pilée. On laisse la température remonter à environ 20°C, essore, lave par 600 cm3 d'eau 2 fois, 300 cm3 d'éthanol à 5°C et 2 fois 400 cm3 d'éther éthylique à environ 20°C. On obtient 131,58 g de difluoro-6,7 dihydro-3,4 carbostyrile sous forme d'un solide beige fondant à 216°C qui est utilisé tel quel pour les étapes ultérieures.

Le difluoro-3',4' N-chloro-3 propionanilide est préparé de la manière suivante :

A une solution de 125 g de 3,4 difluoroaniline dans 80 cm3 de pyridine et 1,5 litre d'acétone chauffée à une température voisine de 55°C, on ajoute, sous agitation, en 1 heure et demie, 139,16 g de chlorure de l'acide chloro-3 propionique et maintient à cette température pendant 1 heure et demie. Après refroidissement à environ 20°C, la solution est versée, sous agitation, dans un mélange de 1 litre d'eau et 500 g de glace pilée. On laisse la température remonter à environ 20°C et extrait par 3 fois 500 cm3 de dichlorométhane. Les extraits organiques réunis sont lavés par 500 cm3 d'acide chlorhydrique N, 5 fois 500 cm3 d'eau, séchés sur sulfate de magnésium, filtrés et concentrés sous pression réduite (20 kPa) à environ 50°C. Le solide obtenu est repris par 500 cm3 de n-hexane, essoré, lavé par 2 fois 100 cm3 du même solvant. On obtient 202,9 g de difluoro-3',4' N-chloro-3 propionanilide sous forme d'un solide beige fondant à 76°C qui est utilisé sans autre purification pour les étapes ultérieures.

### EXEMPLE 24

Une suspension de 0,93 g d'acide difluoro-7,8 méthoxy-1 oxo-4 dihydro-1,4 benzo [b] naphtyridine-1,8 carboxylique-3 dans 0,6 g de méthyl-1 pipérazine et 20 cm3 de diméthylsulfoxyde est chauffée à une température voisine de 80°C pendant 5 minutes. Après refroidissement à environ 20°C, le mélange réactionnel est versé dans 30 cm3 d'eau, additionné de 1,5 cm3 d'acide méthanesulfonique 2N, essoré, lavé par 3 fois 5 cm3 d'eau. Après recristallisation dans 30 cm3 de diméthylformamide à 30 % d'éthanol, on obtient 0,55 g d'acide fluoro-7 méthoxy-1 (méthyl-4 pipérazinyl-1)-8 oxo-4 dihydro-1,4 benzo [b] naphtyridine-1,8 sous forme d'un solide brun fondant à 270°C.

### EXEMPLE 25

Une suspension de 0,47 g de d'acide cyclopropyl-1 difluoro-7,8 oxo-4 dihydro-1,4 benzo[b]naphtyridine-1,8 carboxylique-3 et de 0,6 g de méthyl-1 pipérazine dans 7 cm3 de diméthylsulfoxyde est chauffée à une température voisine de 80°C pendant 15 minutes. Le mélange réactionnel est versé dans 25 cm³ d'eau, additionné de 9 cm³ d'acide chlorydrique N. Le solide obtenu est essoré, lavé par 3 fois 5 cm³ d'eau. Après 1 recristallisation dans un mélange de 4,5 cm³ d'éthanol et de 4,5 cm³ de diméthylformamide on obtient 0,29 g d'acide cyclopropyl-1 fluoro-7 (méthyl-4 pipérazinyl-1)-8 oxo-4 dihydro-1,4 benzo[b]naphtyridine-1,8 carboxylique-3 sous forme d'un solide jaune fondant 250°C.
l'acide cyclopropyl-1 difluoro-7,8 oxo-4 dihydro-1,4 benzo [b] naphtyridine-1,8 carboxylique-3 peut être préparé de la manière suivante :

Une suspension de 1,95 g de cyclopropyl-1 éthoxycarbonyl-3 difluoro-7,8 oxo-4 dihydro-1,4 benzo [b] naphtyridine-1,8 dans 20 cm3 d'acide chlorhydrique à 17,5 % et 20 cm3 d'acide acétique est chauffée à une température voisine de 100°C pendant 1 heure 30 minutes. Après refroidissement à environ 20°C, le mélange réactionnel est versé dans 100 cm3 d'eau. Le précipité est essoré, lavé par par 3 fois 20 cm3 d'eau. Après 1 recristallisation dans un mélange de 30 cm3 diméthylformamide et de 30 cm3 d'éthanol on obtient 1,31 g d'acide cyclopropyl-1 difluoro-7,8 oxo-4 dihydro-1,4 benzo[b] naphtyridine-1,8 carboxylique-3 sous forme d'un solide jaune fondant à 284-285°C.

Le cyclopropyl-1 éthoxycarbonyl-3 difluoro-7,8 oxo-4 dihydro-1,4 benzo [b] naphtyridine-1,8 est préparé de la manière suivante :

Une suspension sous agitation de 5,27 g de (chloro-2 difluoro-6,7 quinoléinecarbonyle-3)-2 cyclopropylamino-3 acrylate d'éthyle, dans 2,22 g de diaza-1,8 bicyclo [5,4,0] undécène-7 (DBU) et 120 cm3 d'éthanol est chauffée à une température voisine de 75°C pendant 35 minutes. Après refroidissement à environ 20°C, le mélange réactionnel es repris par 100 cm3 d'eau, extrait par 1 fois 100 cm3 et 2 fois 50 cm3 de trichlorométhane. Les extraits organiques réunis sont lavés par 3 fois 50 cm3 d'eau, séchés sur sulfate de magnésium, filtrés et concentrés sous pression réduite (20 kPa) à une température voisine de 20°C. L'extrait sec obtenue est repris par 30 cm3 d'éther isopropylique éssoré et recristallisé dans un mélange de 75 cm3 d'éthanol et de 75 cm3 de diméthylformamide. On obtient 3,57 g de cyclopropyl-1 éthoxycarbonyl-3 difluoro-7,8 oxo-4 dihydro-1,4 benzo [b] naphtyridine-1,8 sous forme d'un solide jaune fondant à 229-230°C.

Le (chloro-2 difluoro-6,7 quinoléinecarbonyle-3)-2 cyclopropylamino-3 acrylate d'éthyle est préparé de la manière suivante :

Une solution de 6,25 g de (chloro-2 difluoro-6,7 quinoléinecarbonyle-3)-2 diméthylamino-3 acrylate d'éthyle dans 2,91 g de cyclopropylamine et 25 cm3 de trichlorométhane est agitée pendant 3 heures à une température voisine de 20°C. Le mélange réactionnel est concentré sous pression réduite (20 kPa) à une température voisine de 50°C. L'extrait sec est repris par 50 cm3 d'éther isopropylique, essoré, puis lavé par 20 cm3 du même solvant.

On obtient 5,27 g de (chloro-2 difluoro-6,7 quinoléinecarbonyle-3)-2 cyclopropylamino-3 acrylate d'éthyle sous forme d'un solide orangé fondant à 116-117°C. Ce produit est utilisé sans autre purification pour les étapes ultérieures.

### EXEMPLE 26

Une suspension de 4 g d'acide trifluoro-7,8,9 méthyl-1 oxo-4 dihydro-1,4 benzo [b] naphtyridine-1,8 carboxylique-3 dans 60 cm3 de diméthylsulfoxyde et 3 g de méthyl-1 pipérazine est chauffée à 80°C pendant 1 heure et demie. Après refroidissement à environ 20°C, on ajoute 150 cm3 d'eau. La solution obtenue est additionnée de 18 cm3 d'acide acétique à 10 %. Le précipité formé est essoré, lavé par 3 fois 50 cm3 d'eau et recristallisé dans 50 cm3 de diméthylformamide. On obtient 4 g d'acide difluoro-7,9 méthyl-1 (méthyl-4 pipérazinyl-1)-8 oxo-4 dihydro-1,4 benzo [b] naphtyridine-1,8 carboxylique-3 sous forme d'un solide jaune fondant à 316°C.

L'acide trifluoro-7,8,9 méthyl-1 oxo-4 dihydro-1,4 benzo [b]naphtyridine-1,8 carboxylique-3 est préparé de la manière suivante :

Une suspension de 4 g d'éthoxycarbonyl-3 trifluoro-7,8,9 méthyl-1 oxo-4 dihydro-1,4 benzo [b] naphtyridine-1,8 dans 30 cm3 d'acide acétique et 30 cm3 d'acide chlorhydrique à 50 % est chauffée à une température voisine de 100°C pendant 2 heures. Après refroidissement à environ 20°C, on ajoute 100 cm3 d'eau. Le précipité formé est essoré, lavé par 3 fois 50 cm3 d'eau et recristallisé dans 80 cm3 de diméthylformamide. On obtient 3,4 g d'acide trifluoro-7,8,9 méthyl-1 oxo-4 dihydro-1,4 benzo [b] naphtyridine-1,8 carboxylique-3 sous forme d'un solide incolore fondant à 350-352°C.

L'éthoxycarbonyl-3 trifluoro-7,8,9 méthyl-1 oxo-4 dihydro-1,4 benzo [b] naphtyridine-1,8 est préparé de la manière suivante :

Dans une suspension sous agitation de 19,3 g de (chloro-2 trifluoro-6,7,8 quinoléinecarbonyle-3)-2 diméthylamino-3 acrylate d'éthyle dans 150 cm3 d'éthanol maintenue à une température voisine de 5°C, on ajoute en 10 minutes entre 5 et 10°C, une solution à environ 5°C de 10 g de méthylamine dans 50 cm3 d'éthanol, agite 1 heure entre 5 et 10°C et laisse la température remonter à environ 20°C. La solution obtenue est additionnée de 7,6 g de D.B.U. et chauffée à environ 30°C pendant 1 heure. Après refroidissement à une température voisine de 20°C, le produit est essoré, lavé par 2 fois 100 cm3 d'éthanol et 2 fois 100 cm3 d'éther isopropylique. On obtient 13,4 g d'éthoxycarbonyl-3 trifluoro-7,8,9 méthyl-1 oxo-4 dihydro-1,4 benzo [b] naphtyridine-1,8 sous forme d'un solide jaune fondant à 320°C qui est utilisé sans autre purification pour les étapes ultérieures.

Le (chloro-2 trifluoro-6,7,8 quinoléinecarbonyle-3)-2 diméthylamino-3 acrylate d'éthyle peut être préparé de la manière suivante :

Une suspension de 26,7 g de (chloro-2 trifluoro-6,7,8 quinolyl-3)-3 oxo-3 propionate d'éthyle dans 270 cm3 d'acétate d'éthyle et 32 cm3 de N,N diméthylformamidediméthylacétal est chauffée à une température voisine de 75°C, sous agitation, pendant 2 heures. Le mélange réactionnel est concentré à sec sous pression réduite (20kPa) à environ 50°C. L'extrait sec est repris par 175 cm3 d'éther isopropylique, essoré, lavé par 2 fois 85 cm3 du même solvant. On obtient 19,32 g de (chloro-2 trifluoro-6,7,8 quinoléinecarbonyle-3)-2 diméthylamino-3 acrylate d'éthyle sous forme d'un solide orange fondant à 118°C qui est utilisé sans autre purification pour les étapes ultérieures.

Le (chloro-2 trifluoro-6,7,8 quinolyl-3)-3 oxo-3 propionate d'éthyle est préparé de la manière suivante :

Une suspension de 46,3 g d'acide chloro-2 trifluoro-6,7,8 quinoléine carboxylique-3 dans 640 cm3 de trichlorométhane et 84 cm3 de chlorure de thionyle est chauffée, sous agitation, à une température voisine de 60°C pendant 6 heures. La solution obtenue est concentrée à sec sous pression réduite (20kPa) à environ 50°C. L'extrait sec obtenu est repris par 140 cm3 d'éther de pétrole (40-60), essoré, lavé par 2 fois 60 cm3 du même solvant. Les 47,61 g du solide jaune obtenu sont mis en solution dans 400 cm3 de tétrahydrofuranne. Cette solution est introduite, goutte à goutte, sous agitation, en 1 heure et demie, entre 5 et 10°C,m dans 250 cm3 d'une solution de chélate de magnésium du monomalonate d'éthyle dans le tétrahydrofuranne préparé dans les conditions de l'exemple 23. On laisse la température remonter à environ 20°C et agite encore 2 heures dans ces conditions. La solution obtenue, est introduite, goutte à goutte, sous forte agitation, en 1 heure, à une température voisine de 5°C dans 1750 cm3 d'acide sulfurique 0,5 N. On agite encore 2 heures à cette température, extrait à environ 5°C par 3 fois 600 cm3 d'éther éthylique. Les phases organiques réunies sont lavées par 3 fois 500 cm3 d'eau, séchées sur sulfate de magnésium, et concentrées sous pression réduite (20kPa) à une température voisine de 30°C. L'extrait sec est repris par un mélange de 135 cm3 d'éther ispropylique et de 15 cm3 de n-hexane, essoré à environ 5°C, lavé par 2 fois 115 cm3 du même mélange à la même température. On obtient 47,4 g de (chloro-2 trifluoro-6,7,8 quinolyl-3)-3 oxo-3 propionate d'éthyle sous forme d'un solide beige fondant à 78-80°C qui est utilisé sans autre purification pour les étapes ultérieures.

L'acide chloro-2 trifluoro-6,7,8 quinoléine carboxylique-3 est préparé de la manière suivante :

A une suspension, sous agitation, refroidie à environ 10°C, de 45,7 g de chloro-2 trifluoro-6,7,8 formyl-3 dihydro-1,4 quinoléine dans 585 cm3 de potasse N, on ajoute en 1 heure en maintenant la température entre 10 et 14°C, une solution de 69,65 g de permanganate de potassium dans 730 cm3 d'eau. On laisse encore agiter 30 minutes à environ 10°C. On ajoute 12 g de dithionite de sodium, agite 10 minutes à une température voisine de 10°C, filtre sur silice diatomée et lave par 3 fois 400 cm3 d'eau. Le filtrat et les lavages sont réunis et additionnés de 70 cm3 d'acide chlorhydrique en solution aqueuse à 35 %. Le précipité formé est extrait par 3 fois 500 cm3 d'acétate d'éthyle. Les extraits organiques réunis sont séchés sur sulfate de magnésium, filtrés et concentrés sous pression réduite (20kPa) à 50°C. Le résidu est repris par un mélange de 100 cm3 d'éther éthylique et 100 cm3 d'éther isopropylique, essoré, lavé par 100 cm3 du même mélange. On obtient 46,43 g d'acide chloro-2 trifluoro-6,7,8 quinoléine carboxylique-3 sous forme d'un solide incolore se décomposant à 225-230°C qui est utilisé sans autre purification pour les étapes ultérieures.

La chloro-2 trifluoro-6,7,8 formyl-3 dihydro-1,4 quinoléine est préparée de la manière suivante :

A un mélange de 525 cm3 de trichlorométhane et de 49 cm3 de diméthylformamide, on ajoute en 40 minutes, sous agitation, entre 5 et 10°C, 50 cm3 de chlorure de phosphoryle, agite 15 minutes à cette température et laisse la température remonter à environ 20°C. A la solution obtenue, on ajoute en 20 minutes à environ 20°C, progressivement, sous forte agitation, 46,8 g de trifluoro-6,7,8 dihydro-3,4 carbostyrile. On laisse agiter 30 minutes à une température voisine de 20°C, chauffe à environ 60°C et maintient à cette même température pendant 2 heures et demie. Le mélange réactionnel est concentré sous pression réduite (20kPa) à environ 50°C. Le résidue huileux est versé sous forte agitation dans 500 g de glace. On ajoute, par petites fractions, en 30 minutes, 100 g d'acétate de sodium. La suspension obtenue est versée en 15 minutes, sous forte agitation dans 1 litre d'eau préalablement chauffée à environ 90°C et agite encore 15 minutes à cette même température. L'insoluble est essoré à environ 90°C et lavé par 3 fois 250 cm3 d'eau. On obtient 47,7 g de chloro-2 trifluoro-6,7,8 formyl-3 dihydro-1,4 quinoléine sous forme d'un solide incolore se décomposant à 220°C.

Le trifluoro 6,7,8 dihydro-3,4 carbostyrile est préparé de la manière suivante :

24,35 g de trifluoro-6,7,8 carbostyrile en suspension dans un mélange de 450 cm3 d'éthanol et 150 cm3 de diméthylformamide sont hydrogénés, sous agitation, à environ 50°C, en présence de 5 g de nickel de Raney sous une pression de 1 atmosphère, jusqu'à fin d'absorption d'hydrogène. Le nickel de Raney utilisé de qualité W-2 est préalablement lavé par 50 cm3 d'une solution d'acide acétique aqueux à 2 %, par 2 fois 50 cm3 d'eau et par 3 fois 50 cm3 d'éthanol. Le mélange réactionnel est additionné de 250 cm3 de diméthylformamide, filtré à environ 50°C sur silice diatomée. Le filtrat est concentré sous pression réduite (20kPa) à environ 70°C. L'extrait sec est repris par 150 cm3 d'eau, essoré et lavé par 2 fois 50 cm3 d'eau. On obtient 23,6 g de trifluoro 6,7,8 dihydro-3,4 carbostyrile sous forme d'un solide beige clair fondant à 217°C qui est utilisé sans autre purification pour les étapes ultérieures.

Le trifluoro-6,7,8 carbostyrile est préparé de la manière suivante :

60,83 g de chloro-4 trifluoro-6,7,8 carbostyrile en suspension dans 520 cm3 d'acide acétique et 38,15 cm3 de triéthylamine sont hydrogénés sous une pression de 1 atmosphère, en présence de 5,25 g de palladium sur charbon à 10 % jusqu'à fin d'absorption d'hydrogène, à une température voisine de 25°C. Le mélange réactionnel est ensuite chauffé à environ 40°C, filtré à cette même température, sur silice diatomée pour filtration. Le filtrat est concentré sous pression réduite (20kPa) à une température voisine de 50°C. L'extrait sec est repris par 400 cm3 d'eau ; l'insoluble est essoré, lavé par 4 fois 170 cm3 d'eau, 2 fois 110 cm3 d'éthanol et 2 fois par 100 cm3 d'éther isopropylique. On obtient 48,35 g de trifluoro-6,7,8 carbostyrile, sous forme d'un solide incolore se sublimant à 288°C, qui est utilisé sans autre purification pour les étapes ultérieures.

Le chloro-4 trifluoro-6,7,8 carbostyrile est préparé de la manière suivante :

Une suspension de 70,4 g de chloro-4 éthoxy-2 trifluoro-6,7,8 quinoléine dans 170 cm3 d'une solution aqueuse d'acide chlorhydrique à 35 %, 420 cm3 d'acide acétique et 250 cm3 d'eau est chauffée sous agitation à une température voisine de 100°C pendant 2 heures et demie. Après refroidissement à environ 20°C, le mélange réactionnel est versé dans 1100 cm3 d'eau à environ 5°C, agité 15 minutes à cette même température, puis l'insoluble est essoré et lavé par 3 fois 220 cm3 d'eau. On obtient 61 g de chloro-4 trifluoro-6,7,8 carbostyrile, sous forme d'un solide crème fondant à 213°C qui est utilisé sans autre purification pour les étapes ultérieures.

La chloro-4 éthoxy-2 trifluoro-6,7,8 quinoléine est préparée de la manière suivante :

Une suspension de 69,5 g d'éthoxy-2 trifluoro-6,7,8 hydroxy-4 quinoléine dans 430 cm3 de chlorure de phosphoryle est chauffée, sous agitation à une température voisine de 100°C pendant 30 minutes. La solution obtenue est concentrée, sous pression réduite (20kPa) à environ 60°C, jusqu'à un volume de 100 cm3. Le résidu est repris par 750 cm3 d'acétate d'éthyle ; la solution obtenue est versée sous agitation, en 10 minutes, dans un mélange de 400 cm3 d'eau et 200 g de glace et laisse agiter dans ces conditions pendant 30 minutes. Après séparation de l'extrait organique, la phase aqueuse est de nouveau extraite par 2 fois 250 cm3 d'acétate d'éthyle. Les extraits organiques réunis sont lavés par 3 fois 250 cm3 d'eau, séchés sur sulfate de magnésium et concentrés sous pression réduite (20kPa) à environ 40°C. Le résidu huileux obtenu est repris par 370 cm3 d'éther de pétrole ((40-60). Après filtration sur silice diatomée, le filtrat est concentré à sec, sous pression réduite (20kPa) à environ 30°C. On obtient 70,7 g de chloro-4 éthoxy-2 trifluoro-6,7,8 quinoléine, sous forme d'un solide beige fondant à 45°C qui est utilisé sans autre purification pour les étapes ultérieures.

L'éthoxy-2 trifluoro-6,7,8 hydroxy-4 quinoléine peut être préparée de la manière suivante :

Une solution de 122 g de trifluoro-2,3,4 N[(éthoxy-1' éthoxycarbonyl-2') éthylidène]-aniline dans 120 cm3 d'oxyde de phényle est introduite, goutte à goutte, en 25 minutes, sous agitation, dans 600 cm3 d'oxyde de phényle à une température voisine de 250°C tout en éliminant l'éthanol formé par distillation. Après agitation pendant 15 minutes à cette même température, la solution est refroidie à environ 20°C et additionnée de 750 cm3 de n-hexane. Le précipité formé est essoré et lavé 3 fois par 200 cm3 de n-hexane. On obtient 69,5 g d'éthoxy-2 trifluoro-6,7,8 hydroxy-4 quinoléine sous forme d'un solide beige fondant à 171°C qui est utilisé sans autre purification pour les étapes ultérieures.

La trifluoro-2,3,4 N-[(éthoxy-1' éthoxycarbonyl-2') éthylidène]-aniline peut être préparée de la manière suivante :

A une solution de 90 g de chlorhydrate d'(éthoxycarbonyl-2 éthoxy-1) éthylidèneamine dans 820 cm3 d'éthanol, on ajoute en une seule fois, sous agitation, 58,8 g de trifluoro-2,3,4 aniline. Après 48 heures d'agitation à une température voisine de 20°C, la suspension obtenue est filtrée ; le filtrat est concentré sous pression réduite (20kPa) à une température voisine de 50°C. Le résidu huileux est repris par 250 cm3 d'eau. Le mélange obtenu est extrait par 3 fois 200 cm3 d'éther éthylique. Les extraits organiques réunis sont lavés par 4 fois 150 cm3 d'eau, séchés sur sulfate de magnésium et concentrés sous pression réduite (20kPa) à environ 30°C. On obtient 122 g de trifluoro-2,3,4 N-[(éthoxy-1' éthoxycarbonyl-2') éthylidène]-aniline sous forme d'une huile jaune qui est utilisée sans autre purification pour les étapes ultérieures.

Le chlorhydrate d'(éthoxycarbonyl-2 éthoxy-1) éthylidèneamine a été préparé selon la méthode décrite par A. Pinner, et coll., Ber. Dtsch. Chem. Ges., 28, 478 (1895).

### EXEMPLE 27

Une suspension de 2 g d'acide trifluoro-6,7,8 méthoxy-1 oxo-4 dihydro-1,4 benzo [b] naphtyridine-1,8 carboxylique-3 et de 2,8 g de pipérazine dans 40 cm3 de diméthylsulfoxyde est chauffée, sous agitation à environ 50°C pendant 45 minutes. Après refroidissement à une température voisine de 20°C, la suspension obtenue est versée dans 100 cm3 d'eau, additionnée de 9,22 g d'acide méthanesulfonique. Un léger insoluble est éliminé par filtration sursilice diatomée. Le filtrat est additionné de 32 cm3 de potasse aqueuse 2N. Le précipité obtenu est essoré, lavé par 3 fois 50 cm3 d'eau et recristallisé dans 80 cm3 de diméthylformamide. On obtient 1,4 g d'acide difluoro-7,9 méthoxy-1 oxo-4 (pipérazinyl-1)-8 dihydro-1,4 benzo[b]naphtyridine-1,8 carboxylique-3 sous forme d'un solide jaune fondant à 305-308°C.

L'acide trifluoro-6,7,8 méthoxy-1 oxo-4 dihydro-1,4 benzo [b] naphtyridine-1,8 carboxylique-3 a été préparé dans les conditions de l'exemple 25, mais à partir de 9 g d'éthoxycarbonyl-3 trifluoro-6,7,8 méthoxy-1 oxo-4 dihydro-1,4 benzo[b]naphtyridine-1,8. On obtient 7,7 g d'acide trifluoro-6,7,8 méthoxy-1 oxo-4 dihydro-1,4 benzo [b] naphtyridine-1,8 carboxylique-3 sous forme d'un solide beige fondant à 322°C.

L'éthoxycarbonyl-3 trifluoro-6,7,8 méthoxy-1 oxo-4 dihydro-1,4 benzo [b] naphtyridine-1,8 a été préparé dans les conditions suivantes:

Une suspension de 5,1g de chlorhydrate de méthylhydroxylamine dans 120 cm3 de trichlorométhane est additionnée de 8,7 cm3 de triéthylamine. A la solution obtenue, on ajoute à environ 20°C, 7,8 g de (chloro-2 trifluoro-6,7,8 quinoléinecarbonyle-3)-2 diméthylamino-3 acrylate d'éthyle. Après 2 heures d'agitation à cette température, la solution est concentrée à sec, sous pression réduite (20kPa) à environ 50°C. Le résidu obtenu est repris par 150 cm3 d'éthanol et 10cm3 de triéthylamine et chauffé, sous agitation, pendant 30 minutes. Après refroidissement à environ 20°C, l'insoluble est essoré, lavé par 3 fois 50 cm3 d'éthanol et 2 fois 50 cm3 d'éther isopropylique. Après recristallisation dans 120 cm3 de diméthylformamide, on obtient 9 g d'éthoxycarbonyl-3 trifluoro-6,7,8 méthoxy-1 oxo-4 dihydro-1,4 benzo[b]naphtyridine-1,8 sous forme d'un solide jaune fondant à 298-300°C.

### EXEMPLE 28

Une solution de 1,15 g d'acide fluoro-7 méthyl-1 (méthyl-3 pipérazinyl-1)-8 oxo-4 dihydro-1,4 benzo[b]naphtyridine-1,8 carboxylique-3 dans 1,35 cm3 d'acide formique à 98 % et 3,25 cm3 de formaldéhyde en solution aqueuse à 30 % est chauffée à une température voisine de 100°C pendant 2 heures. Le mélange réactionnel est concentré sous pression réduite (20kPa) à 50°C puis additionné de 5 cm3 d'eau, la solution obtenue est amenée à pH 7 par addition de 0,5 cm3 de potasse aqueuse 2 N et chauffée à une température voisine de 100°C pendant 2 minutes. Le produit qui cristallise est essoré à 20°C, lavé par 2 fois 10 cm3 d'eau. Le produit brut obtenu est recristallisé 2 fois dans 10 cm3 de diméthylformamide à chaque fois. On obtient 0,55 g d'acide (diméthyl-3,4 pipérazinyl-1)-8 fluoro-7 méthyl-1 oxo-4 dihydro-1,4 benzo[b]naphtyridine-1,8 carboxylique-3 sous forme d'un solide jaune fondant à 306-308°C.

### EXEMPLE 29

En opérant dans les conditions de l'exemple 28, mais à partir de 2,3 g d'acide éthyl-1 fluoro-7 (méthyl-3 pipérazinyl-1)-8 oxo-4 dihydro-1,4 benzo[b]naphtyridine-1,8 carboxylique-3, de 2,26 cm3 d'acide formique à 98 % et de 5,6 cm3 de formaldéhyde en solution aqueuse à 30 %, on obtient 1,75 g d'acide (diméthyl-3,4 pipérazinyl-1)-8 éthyl-1 fluoro-7 oxo-4 dihydro-1,4 benzo[b]naphtyridine-1,8 carboxylique-3 sous forme d'un solide jaune fondant à 293-294°C.

### EXEMPLE 30

On opère dans les conditions de l'exemple 28 mais à partir de 1,9 g d'acide cyclopropyl-1 fluoro-7 (méthyl-3 pipérazinyl-1)-8 oxo-4 dihydro-1,4 benzo[b]naphtyridine-1,8 carboxylique-3, de 1,38 cm3 d'acide formique et de 3,30 cm3 de formaldéhyde en solution aqueuse à 30%. Après une recristallisation du produit brut dans 50 cm3 d'éthanol, on obtient 1,3 g d'acide cyclopropyl-1 (diméthyl-3,4 pipérazinyl-1)-8 fluoro-7 oxo-4 dihydro-1,4 benzo[b]naphtyridine-1,8 carboxylique-3 sous forme d'un solide jaune fondant à 219°C.

### EXEMPLE 31

Une suspension de 0,85 g de cyclopropyl-1 éthoxycarbonyl-3 fluoro-7 (méthyl-4 pipérazinyl-1)-8 oxo-4 dihydro-1,4 benzo[b]naphtyridine-1,8 dans 12 cm³ d'éthanol, 5 cm³ de potasse acqueuse 2 N et 7 cm³ d'eau est chauffée à une température voisine de 80°C pendant 1 heure. Après addition de 5,8 cm³ d'une solution acqueuse à 10 % d'acide acétique, le précipité obtenu est essoré, lavé par 3 fois 5 cm³ d'eau. Après 2 recristallisations dans un mélange de 7,5 cm³ d'éthanol et de 7,5 cm³ de diméthylformamide à chaque fois, on obtient 0,4 g d'acide cyclopropyl-1 fluoro-7 (méthyl-4 pipérazinyl-1)-8 oxo-4 dihydro-1,4 benzo[b]naphtyridine-1,8 carboxylique-3 sous forme d'un solide jaune fondant à 250°C.

La cyclopropyl-1 éthoxycarbonyl-3 fluoro-7 (méthyl-4 pipérazinyl-1)-8 oxo-4 dihydro-1,4 benzo[b]naphtyridine-1,8 peut être préparée de la manière suivante :

Une suspension de 1,2 g de cyclopropyl-1 éthoxycarbonyl-3 difluoro-7,8 oxo-4 dihydro-1,4 benzo[b]naphtyridine-1,8 1,4 g de méthyl-1 pipérazine et 20 cm³ de diméthylsulfoxyde est chauffée à une température voisine de 95°C pendant 45 minutes. Après refroidissement à une température voisine de 20°C, le mélange réactionnel est dilué par 100 cm³ d'eau, extrait par 3 fois 30 cm³ de trichlorométhane. Les extraits organiques réunis sont lavés par 3 fois 30 cm³ d'eau, séchés sur sulfate de magnésium, filtrés et concentrés sous pression réduite (20 kPa) à une température voisine de 50°C. Le solide obtenu est repris par 20 cm³ d'éther isopropylique, essoré, lavé par 10 cm³ du même solvant et recristallisé dans 150 cm³ d'acétate d'éthyl. On obtient 1,1 g de cyclopropyl-1 éthoxycarbonyl-3 fluoro-7 (méthyl-4 pipérazinyl-1)-8 oxo-4 dihydro-1,4 benzo[b]naphtyridine-1,8 sous forme d'un solide jaune fondant à 223°C.

### EXEMPLE 32

Une suspension de 0,98 g d'éthoxycarbonyl-3 fluoro-7 méthoxy-1 (méthyl-4 pipérazinyl-1)-8 oxo-4 dihydro-1,4 benzo [b] naphtyridine-1,8 dans 15 cm3 d'éthanol, 9 cm3 d'eau et 6 cm3 de potasse aqueuse 2N est agitée à une température voisine de 20°C pendant 1 heure. La solution obtenue est additionnée de 6 cm3 d'acide méthanesulfonique 2N, extraite par 3 fois 10 cm3 de trichlorométhane. Les extraits organiques réunis sont lavés par 3 fois 5 cm3 d'eau, séchés sur sulfate de magnésium, filtrés et concentrés à sec sous pression réduite (20 kPa) à environ 50°C. L'extrait sec obtenu est repris par 5 cm3 d'éther isopropylique, essoré, lavé par 2 fois 2 cm3 du même solvant. Après 1 recristallisation dans 30 cm3 de diméthylformamide à 30 % d'éthanol, on obtient 0,3 g d'acide fluoro-7 méthoxy-1 (méthyl-4 pipérazinyl-1)-8 oxo-4 dihydro-1,4 benzo[b]naphtyridine-1,8 carboxylique-3 sous forme d'un solide brun fondant à 270°C.

L'éthoxycarbonyl-3 fluoro-7 méthoxy-1 (méthyl-4 pipérazinyl-1)-8 oxo-4 dihydro-1,4 benzo [b] naphtyridine-1,8 est préparé dans les conditions suivantes :

Une suspension de 1,17 g d'éthoxycarbonyl-3 difluoro-7,8 méthoxy-1 oxo-4 dihydro-1,4 benzo[b] naphtyridine-1,8 et de 0,7 g de méthyl-1 pipérazine dans 15 cm3 de diméthylsulfoxyde est chauffée à une température voisine de 95°C pendant 30 minutes. Après refroidissement à une température voisine de 20°C, le mélange réactionnel est versé dans 60 cm3 d'eau, extrait par 3 fois 25 cm3 de trichlorométhane. Les extraits organiques réunis sont lavés par 3 fois 25 cm3 d'eau, séchés sur sulfate de magnésium, filtrés et concentrés sous pression réduite (20 kPa) à une température voisine de 50°C. Le solide obtenu est repris par 10 cm3 d'éther isopropylique, essoré, lavé par 5 cm3 du même solvant. Après recristallisation dans 90 cm3 d'éthanol, on obtient 1,18 g d'éthoxycarbonyl-3 fluoro-7 méthoxy-1 (méthyl-4 pipérazinyl-1)-8 oxo-4 dihydro-1,4 benzo [b] naphtyridine-1,8 sous forme d'un solide jaune fondant à 230°C.

### EXEMPLE 33

En opérant dans les conditions de l'exemple 35 ci-après, mais à partir de 2 g d'éthoxycarbonyl-3 difluoro-7,9 méthyl-1 oxo-4 (perhydrodiazépin-1,4 yl-1)-8 dihydro-1,4 benzo[b]naphtyridine-1,8, on obtient 1,5 g d'acide difluoro-7,9 méthyl-1 oxo-4 (perhydrodiazépin-1,4 yl-1)-8 dihydro-1,4 benzo[b]naphtyridine-1,8 carboxylique-3 sous forme d'un solide jaune fondant à 340-342°C.

L'éthoxycarbonyl-3 difluoro-7,9 méthyl-1 oxo-4 (perhydrodiazépin-1,4 yl-1)-8 dihydro-1,4 benzo[b]naphtyridine-1,8 a été préparée dans les conditions de l'exemple 3, mais à partir de 2 g d'éthoxycarbonyl-3 trifluoro-7,8,9 méthyl-1 oxo-4 dihydro-1,4 benzo [b] naphtyridine-1,8 et de 2 g d'hexahydrodiazépine-1,4. On obtient 2 g d'éthoxycarbonyl-3 difluoro-7,9 méthyl-1 oxo-4 (perhydrodiazépin-1,4 yl-1)-8 dihydro-1,4 benzo[b]naphtyridine-1,8 sous forme d'un solide jaune fondant à 226°C.

### EXEMPLE 34

Une suspension de 0,8 g d'éthoxycarbonyl-3 difluoro-7,9 méthyl-1 oxo-4 (pipérazinyl-1)-8 dihydro-1,4 benzo[b]naphtyridine-1,8 dans 20 cm3 d'éthanol et 20 cm3 de potasse aqueuse N est chauffée à une température voisine de 75°C pendant 1 heure et demie. La solution obtenue est additionnée à cette température de 12 g d'une solution aqueuse d'acide acétique à 10%. L'insoluble obtenu est essoré à environ 75°C et lavé 3 fois par 30 cm3 d'eau à environ 20°C. Après 1 recristallisation dans 90 cm3 de diméthylformamide, on obtient 0,5 g d'acide difluoro-7,9 méthyl-1 oxo-4 (pipérazinyl-1)-8 dihydro-1,4 benzo[b]naphtyridine-1,8 carboxylique-3 sous forme d'un solide jaune fondant à 345-347°C.

L'éthoxycarbonyl-3 difluoro-7,9 méthyl-1 oxo-4 (pipérazinyl-1)-8 dihydro-1,4 benzo[b]naphtyridine-1,8 a été préparée de la manière suivante:

Une suspension de 2 g d'éthoxycarbonyl-3 trifluoro-7,8,9 méthyl-1 oxo-4 dihydro-1,4 benzo[b]naphtyridine-1,8 dans 30 cm3 de diméthylsulfoxide et 5 g de pipérazine est chauffée à une température voisine de 100°C, sous agitation, pendant 2 heures. La solution obtenue à environ 100°C, est versée, sous agitation, dans un mélange de 150 cm3 d'eau et 50 g de glace. La solution est extraite par 3 fois 40 cm3 de trichlorométhane. Les phases organiques réunies sont extraites par 2 fois 50 cm3 d'acide méthanesulfonique 0,1N. Les phases aqueuses réunies sont additionnées de 10 g de carbonate de potassium. Le mélange est extrait par 3 fois 40 cm3 de trichlorométhane. Les extraits organiques réunis sont lavés par 2 fois 50 cm3 d'eau, séchés sur sulfate de magnésium, filtrés et concentrés à sec, sous pression réduite (20kPa) à environ 50°C. Le résidu obtenu est recristallisé dans 25 cm3 d'éthanol. On obtient 0,8 g d'éthoxycarbonyl-3 difluoro-7,9 méthyl-1 oxo-4 (pipérazinyl-1)-8 dihydro-1,4 benzo [b] naphtyridine-1,8 sous forme d'un solide jaune fondant à 221°C.

### EXEMPLES 35

Une suspension de 1,5 g de d'éthoxycarbonyl-3 difluoro-7,9 méthyl-1 (méthyl-3 pipérazinyl-1)-8 oxo-4 dihydro-1,4 benzo [b] naphtyridine-1,8-(RS) dans 20 cm3 d'éthanol et 20 cm3 de potasse aqueuse N est chauffée à une température voisine de 75°C pendant 1 heure et demie. La solution obtenue est additionnée à cette dernière température de 12 g d'une solution aqueuse d'acide acétique à 10 %. L'insoluble obtenu est essoré à environ 75°C et lavé 3 fois par 30 cm3 d'eau à environ 20°C. Après 1 recristallisation dans 100 cm3 de diméthylformamide, on obtient 0,9 g d'acide difluoro-7,9 méthyl-1 (méthyl-3 pipérazinyl-1)-8 oxo-4 dihydro-1,4 benzo[b]naphtyridine-1,8 carboxylique-3-(RS) sous forme d'une solide jaune fondant à 380-382°C.

L'éthoxycarbonyl-3 difluoro-7,9 méthyl-1 (méthyl-3 pipérazinyl-1)-8 oxo-4 dihydro-1,4 benzo[b]naphtyridine-1,8-(RS) a été préparée de la manière suivante:

Une suspension de 2 g d'éthoxycarbonyl-3 trifluoro-7,8,9 méthyl-1 oxo-4 dihydro-1,4 benzo [b] naphtyridine-1,8 dans 30 cm3 de diméthylsulfoxyde et 5 g de méthyl-2 pipérazine est chauffée à une température voisine de 100°C, sous agitation, pendant 2 heures. A cette même température, la solution obtenue est versée, sous agitation, dans un mélange de 150 cm3 d'eau et 50 g de glace. On ajoute à environ 20°C, 5 g de carbonate de potassium et extrait par 3 fois 5 cm3 de trichlorométhane. Les extraits organiques réunis sont lavés par 2 fois 50 cm3 d'eau, séchés sur sulfate de magnésium, filtrés et concentrés à sec sous pression réduite (20kPa) à environ 50°C. La résidu obtenu est recristallisé dans 30 cm3 d'éthanol. On obtient 1,6 g d'éthoxycarbonyl-3 difluoro-7,9 méthyl-1 (méthyl-3 pipérazinyl-1)-8 oxo-4 dihydro-1,4 benzo [b] naphtyridine-1,8-(RS) sous forme d'une solide jaune fondant à 240°C.

### EXEMPLE 36

En opérant dans les conditions de l'exemple 35, mais à partir de 1 g d'éthoxycarbonyl-3 éthyl-1 difluoro-7,9 oxo-4 (pipérazinyl-1)-8 dihydro-1,4 benzo [b] naphtyridine-1,8, on obtient 0,7 g d'acide éthyl-1 difluoro-7,9 oxo-4 (pipérazinyl-1)-8 dihydro-1,4 benzo [b] naphtyridine-1,8 carboxylique-3 sous forme d'un solide jaune fondant à 340-342°C.

L'éthoxycarbonyl-3 éthyl-1 difluoro-7,9 oxo-4 (pipérazinyl-1)-8 dihydro-1,4 benzo [b] naphtyridine-1,8 a été préparée dans les conditions de l'exemple 35 mais à partir de 1,75 g d'éthoxycarbonyl-3 éthyl-1 trifluoro-7,8,9 oxo-4 dihydro-1,4 benzo[b] naphtyridine-1,8 et de 4,3 g de pipérazine, on obtient 1,1 g d'éthoxycarbonyl-3 éthyl-1 difluoro-7,9 oxo-4 (pipérazinyl-1)-8 dihydro-1,4 benzo [b]naphtyridine-1,8 sous forme d'un solide jaune fondant à 229°C.

### EXEMPLE 37

En opérant dans les conditions de l'exemple 35, mais à partir de 1,1 g d'éthoxycarbonyl-3 éthyl-1 difluoro-7,9 (méthyl-4 pipérazinyl-1)-8 oxo-4 dihydro-1,4 benzo [b] naphtyridine-1,8, on obtient 0,75 g d'acide difluoro-7,9, éthyl-1 (méthyl-4 pipérazinyl-1)-8 oxo-4 dihydro-1,4 benzo [b] naphtyridine-1,8 carboxylique-3 sous forme d'un solide jaune fondant à 308°C.

L'éthoxycarbonyl-3 éthyl-1 difluoro-7,9 (méthyl-4 pipérazinyl-1)-8 oxo-4 dihydro-1,4 benzo [b] naphtyridine-1,8 a été préparée dans les conditions de l'exemple 3 mais à partir de 1,4 g d'éthoxycarbonyl-3 éthyl-1 trifluoro-7,8,9 oxo-4 dihydro-1,4 benzo [b] naphtyridine-1,8 et de 4 g de méthyl-1 pipérazine. On obtient 1,1 g d'éthoxycarbonyl-3 éthyl-1 difluoro-7,9 (méthyl-4 pipérazinyl-1)-8 oxo-4 dihydro-1,4 benzo [b] naphtyridine-1,8 sous forme d'un solide jaune fondant à 206°C.

L'éthoxycarbonyl-3 éthyl-1 trifluoro-7,8,9 oxo-4 dihydro-1,4 benzo [b] naphtyridine-1,8 a été préparée dans les conditions suivantes:

Dans une suspension sous agitation de 7,1 g de (chloro-2 trifluoro-6,7,8 quinoléinecarbonyle-3)-2 diméthylamino-3 acrylate d'éthyle dans 100 cm3 d'éthanol maintenue à une température voisine de 5°C, on ajoute en 10 minutes entre 5 et 10°C, 4,5 g d'éthylamine, agite 1 heure entre 5 et 10°C et laisse la température remonter à environ 20°C. La solution obtenue est additionnée de 4 g de diaza-1,8-bicyclo [5.4.0] undécène-7 (DBU) et chauffée à une température voisine de 75°C pendant 1 heure et demie. Après refroidissement à une température voisine de 20°C, le produit est essoré, lavé par 2 fois 30 cm3 d'éthanol et 2 fois 50 cm3 d'éther isopropylique. On obtient 4 g d'éthoxycarbonyl-3 éthyl-1 trifluoro-7,8,9 oxo-4 dihydro-1,4 benzo [b] naphtyridine-1,8 sous forme d'un solide crème fondant à 284°C qui est utilisé sans autre purification pour les étapes ultérieures.

### EXEMPLE 38

En opérant dans les conditions de l'exemple 35, mais à partir de 1,7 g de difluoro-7,9 éthoxycarbonyl-3 éthyl-1 oxo-4 (perhydrodiazépin-1,4 yl-1)-8 dihydro-1,4 benzo[b]naphtyridine-1,8, on obtient 1,1 g d'acide éthyl-1 difluoro-7,9 oxo-4 (perhydrodiazépin-1,4 yl-1)-8 dihydro-1,4 benzo[b]naphtyridine-1,8 carboxylique-3 sous forme d'un solide jaune fondant à 274°C.

Le difluoro-7,9 éthoxycarbonyl-3 éthyl-1 oxo-4 (perhydrodiazépin-1,4 yl-1)-8 dihydro-1,4 benzo [b] naphtyridine-1,8 a été préparée dans les conditions de l'exemple 35, mais à partir de 3 g d'éthoxycarbonyl-3 éthyl-1 trifluoro-7,8,9 oxo-4 dihydro-1,4 benzo [b] naphtyridine-1,8 et de 2 g d'hexahydrodiazépine-1,4. On obtient 1,7 g d'éthoxycarbonyl-3 éthyl-1 difluoro-7,9 oxo-4 (perhydrodiazépin-1,4 yl-1)-8 dihydro-1,4 benzo [b] naphtyridine-1,8 sous forme d'un solide jaune fondant à 190°C.

### EXEMPLE 39

On opère dans les conditions de l'exemple 35, mais à partir de 2 g d'éthoxycarbonyl-3 difluoro-7,9 oxo-4 (pipérazinyl-1)-8 tertiobutyl-1 dihydro-1,4 benzo [b] naphtyridine-1,8. Après recristallisation dans 35 cm3 de diméthylformamide et 35 cm3 d'éthanol, on obtient 1 g d'acide difluoro-7,9 oxo-4 (pipérazinyl-1)-8 tertiobutyl-1 dihydro-1,4 benzo[b]naphtyridine-1,8 carboxylique-3 sous forme d'un solide jaune fondant à 318°C.

L'éthoxycarbonyl-3 difluoro-7,9 oxo-4 (pipérazinyl-1)-8 tertiobutyl-1 dihydro-1,4 benzo [b] naphtyridine-1,8 a été préparée dans les conditions de l'exemple 35, mais à partir de 1,9 g d'éthoxycarbonyl-3 trifluoro-7,8,9 oxo-4 tertiobutyl-1 dihydro-1,4 benzo [b] naphtyridine-1,8 et de 4,3 g de pipérazine. Après 1 recristallisation dans 50 cm3 d'éther isopropylique et 10 cm3 de propanol-2, on obtient 2 g d'éthoxycarbonyl-3 difluoro-7,9 oxo-4 (pipérazinyl-1)-8 tertiobutyl-1 dihydro-1,4 benzo [b] naphtyridine-1,8 sous forme d'un solide jaune fondant à 186°C.

L'éthoxycarbonyl-3 trifluoro-7,8,9 oxo-4 tertiobutyl-1 dihydro-1,4 benzo [b] naphtyridine-1,8 a été préparée dans les conditions suivantes:

Dans une solution sous agitation de 11,7 g de ( chloro-2 trifluoro-6,7,8 quinoléinecarbonyle-3)-2 diméthylamino-3 acrylate d'éthyle dans 150 cm3 de trichlorométhane à une température d'environ 20°C, on ajoute à cette même température en 5 minutes, 9,5 g de tertiobutylamine. Après 4 heures d'agitation à environ 20°C, la solution est concentrée à sec sous pression réduite (20kPa) à environ 50°C. Le résidu obtenu est repris par 100 cm3 d'éthanol. A la solution obtenue, on ajoute 5 g de DBU et chauffe à une température voisine de 75°C pendant 3 heures. Après refroidissement à environ 20°C, le précipité obtenu est essoré, lavé par 2 fois 50 cm3 d'éthanol et 2 fois 50 cm3 d'éther isopropylique. On obtient 9,5 g d'éthoxycarbonyl-3 trifluoro-7,8,9 oxo-4 tertiobutyl-1 dihydro-1,4 benzo [b] naphtyridine-1,8 sous forme d'un solide incolore fondant à 229°C qui est utilisé sans autre purification pour les étapes ultérieures.

### EXEMPLE 40

En opérant dans les conditions de l'exemple 35, mais à partir de 1 g de cyclopropyl-1 éthoxycarbonyl-3 difluoro-7,9 oxo-4 (pipérazinyl-1)-8 dihydro-1,4 benzo [b] naphtyridine-1,8, on obtient 0,70 g d'acide cyclopropyl-1 difluoro-7,9 oxo-4 (pipérazinyl-1)-8 dihydro-1,4 benzo [b] naphtyridine-1,8 carboxylique-3 sous forme d'un solide jaune fondant à 305-307°C.

La cyclopropyl-1 éthoxycarbonyl-3 difluoro-7,9 oxo-4 (pipérazinyl-1)-8 dihydro-1,4 benzo [b] naphtyridine-1,8 a été préparée dans les conditions de l'exemple 35 mais à partir de 1,5 g de cyclopropyl-1 éthoxycarbonyl-3 trifluoro-7,8,9 oxo-4 dihydro-1,4 benzo [b] naphtyridine-1,8 et de 2,7 g de pipérazine. Après 1 recristallisation dans 40 cm3 de propanol-2, on obtient 1 g de cyclopropyl-1 éthoxycarbonyl-3 difluoro-7,9 oxo-4 (pipérazinyl-1)-8 dihydro-1,4 benzo [b] naphtyridine-1,8 sous forme d'un solide jaune fondant à 256°C.

### EXEMPLE 41

On opère dans les conditions de l'exemple 35, mais à partir de 1,7 g de cyclopropyl-1 éthoxycarbonyl-3 difluoro-7,9 (méthyl-4 pipérazinyl-1)-8 oxo-4 dihydro-1,4 benzo[b]naphtyridine-1,8. Après recristallisation dans 60 cm3 d'éthanol, on obtient 1,3 g d'acide cyclopropyl-1 difluoro-7,9 (méthyl-4 pipérazinyl-1)-8 oxo-4 dihydro-1,4 benzo [b] naphtyridine-1,8 carboxylique-3 sous forme d'un solide orange fondant à 248°C.

La cyclopropyl-1 éthoxycarbonyl-3 difluoro-7,9 (méthyl-4 pipérazinyl-1)-8 oxo-4 dihydro-1,4 benzo [b] naphtyridine-1,8 a été préparée dans les conditions de l'exemple 35 mais à partir de 1,5 g de cyclopropyl-1 éthoxycarbonyl-3 trifluoro 7,8,9 oxo-4 dihydro-1,4 benzo [b]naphtyridine-1,8 et de 3 g de méthyl-1 pipérazine. On obtient 1,5 g de cyclopropyl-1 éthoxycarbonyl-3 difluoro-7,9 (méthyl-4 pipérazinyl-1)-8 oxo-4 dihydro-1,4 benzo[b]naphtyridine-1,8 sous forme d'un solide jaune fondant à 230°C.

### EXEMPLE 42

On opère dans les conditions de l'exemple 35, mais à partir de 1,9 g de cyclopropyl-1 éthoxycarbonyl-3 difluoro-7,9 (méthyl-3 pipérazinyl-1)-8 oxo-4 dihydro-1,4 benzo[b]naphtyridine-1,8-(RS). Après recristallisation dans un mélange de 20 cm3 de diméthylformamide et de 20 cm3 d'éthanol, on obtient 1,1 g d'acide cyclopropyl-1,4 benzo [b] naphtyridine-1,8 carboxylique-3-(RS) sous forme d'un solide jaune fondant à 309°C.

La cyclopropyl-1 éthoxycarbonyl-3 difluoro-7,9 (méthyl-3 pipérazinyl-1)-8 oxo-4 dihydro-1,4 benzo [b] naphtyridine-1,8-(RS) a été préparée dans les conditions de l'exemple 35 mais à partir de 1,8 g de cyclopropyl-1 éthoxycarbonyl-3 trifluoro-7,8,9 oxo-4 dihydro-1,4 benzo [b] naphtyridine-1,8 et de 2 g de méthyl-2 pipérazine-(RS). Après 1 recristallisation dans 15 cm3 de propanol-2 et 15 cm3 d'éther isopropylique, on obtient 1,9 g de cyclopropyl-1 éthoxycarbonyl-3 difluoro-7,9 (méthyl-3 pipérazinyl-1)-8 oxo-4 dihydro-1,4 benzo [b] naphtyridine-1,8-(RS) sous forme d'un solide jaune fondant à 190°C.

La présente invention concerne également les compositions pharmaceutiques utilisables en médecine humaine ou vétérinaire qui contiennent comme produit actif au moins un produit de formule générale (I) à l'état pur (sous forme libre ou sous forme de sel) ou sous forme d'une association avec un ou plusieurs diluants ou adjuvants compatibles et pharmaceutiquement acceptables. Ces compositions peuvent être utilisées par voie orale, parentérale ou rectale.

Comme compositions solides pour administration orale, peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

Les compositions pour administration rectale sont les suppositoires ou les capsules rectales qui peuvent contenir, outre le produit actif, des excipients tels que le beurre de cacao ou la suppo-cire.

En thérapeutique humaine ou vétérinaire, les compositions selon l'invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 0,2 et 1 g de produit actif deux fois par jour, par voie orale ou parentérale pour un adulte.

L'exemple suivant, donné à titre non limitatif, illustre une composition selon l'invention :

### Exemple

On prépare selon les techniques habituelles des comprimés dosés à 250 mg de produit actif, ayant la composition suivant.
- acide cyclopropyl-1 fluoro-7 (pipérazinyl-1)-8 oxo-4 dihydro-1,4 benzo[b]naphtyridine-1,8 carboxylique-3 250 mg
- amidon 50 mg
- lactose 35 mg
- talc 15 mg

Les produits de formule générale (I) sont également intéressants dans le domaine de l'agrochimie pour les traitements anti-bactériens des plantes et des végétaux. Il est entendu que les compositions à usage agrochimique renfermant un produit de formule générale (I) entrent aussi dans le cadre de la présente invention.

Par ailleurs, les produits de formule générale (I) peuvent également être utilisés comme agents de conservation ou de désinfection des matières organiques ou minérales. Notamment dans l'industrie des colorants, de matières grasses, du papier, du bois, des polymères ou encore dans l'industrie textile, l'industrie alimentaire ou le traitement des eaux. Il est également entendu que les compositions renfermant un produit de formule générale (I) à l'état pur ou sous forme d'association avec des diluants ou adjuvants compatibles, entrent aussi dans le cadre de la présente invention.

## Revendications (Revendications pour l'(les) Etat(s) contractant(s) suivant(s): AT, BE, CH, DE, DK, FR, GB, IT, LI, LU, NL, SE)

1. Un dérivé de la benzo[b]naphtyridine-1,8 caractérisé en ce qu'il répond à la formule générale : 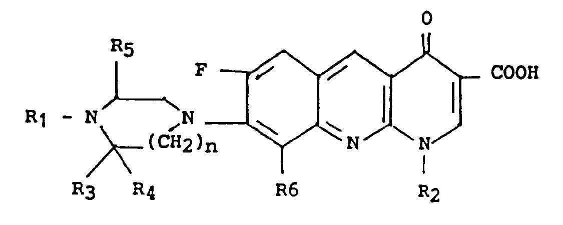 dans laquelle,
soit :
- R₁ représente un atome d'hydrogène ou un radical alcoyle ou hydroxyalcoyle,
- R₂ représente un atome d'hydrogène, un radical alcoyle, fluoroalcoyle, cycloalcoyle, alcoyloxy ou alcoylamino,
- R₃ représente un radical alcoyle et
- R₄ et R₅ sont différents entre eux et représentent un atome d'hydrogène ou un radical alcoyle, ou bien
- R₃ représente un atome d'hydrogène ou un radical alcoyle ou cycloalcoyle et
- R₄ et R₅ sont des atomes d'hydrogène,
- R₆ représente un atome d'hydrogène ou de fluor, et n égal 1 ou 2,
les radicaux alcoyle contenant 1 à 4 atomes de carbone en chaîne droite ou ramifiée, les radicaux cycloalcoyle contenant 3 à 6 atomes de carbone,
ses sels métalliques, ses sels d'addition avec les bases azotées et ses sels d'addition avec les acides ainsi que ses formes hydratées et, le cas échéant ses isomères et leurs mélanges.

2. Un dérivé de la benzonaphtyridine selon la revendication 1, pour lequel
- R₁ représente un atome d'hydrogène ou un radical alcoyle contenant 1 ou 2 atomes de carbone, éventuellement substitué par un radical hydroxy,
- R₂ représente un atome d'hydrogène, un radical alcoyle contenant 1 à 4 atomes de carbone en chaine droite ou ramifiée éventuellement substitué par un atome de fluor
- R₃ représente un radical méthyle et
- R₄ et R₅ qui sont différents entre eux représentent un atome d'hydrogène ou un radical méthyle, ou bien
- R₃ est un atome d'hydrogène ou un radical méthyle et R₄ et R₅ représentent des atomes d'hydrogène,
- R₆ représente un atome d'hydrogène ou de fluor et
- n égale 1 ou 2.
ses sels métalliques, ses sels d'addition avec les bases azotées et ses sels d'addition avec les acides, ainsi que ses formes hydratées et le cas échéant ses isomères et leurs mélanges.

3. Un dérivé selon la revendication 1 constitué par l'acide fluoro-7 méthyl-1 (méthyl-3 pipérazinyl-1)-8 oxo-4 dihydro-1,4 benzo[b]naphtyridine-1,8 carboxylique-3 ainsi que ses sels.

4. Un dérivé selon la revendication 1 constitué par l'acide éthyl-1 fluoro-7 (pipérazinyl-1)-8 oxo-4 dihydro-1,4 benzo[b]naphtyridine-1,8 carboxylique-3 ainsi que ses sels.

5. Un dérivé selon la revendication 1 constitué par l'acide cyclopropyl-1 fluoro-7 (pipérazinyl-1)-8 oxo-4 dihydro-1,4 benzo[b]naphtyridine-1,8 carboxylique-3 ainsi que ses sels.

6. Un dérivé selon la revendication 1 constitué par l'acide difluoro-7,9 méthyl-1 (méthyl-4 pipérazi- nyl-1)-8 oxo-4 dihydro-1,4 benzo[b]naphtyridine-1,8 carboxylique-3 ainsi que ses sels.

7. Un dérivé selon la revendication 1 constitué par l'acide (diméthyl-3,4 pipérazinyl-1)-8 éthyl-1 fluo- ro-7 oxo-4 dihydro-1,4 benzo[b]naphtyridine-1,8 carboxylique-3 ainsi que ses sels.

8. Procédé de préparation d'un dérivé de la benzo[b]naphtyridine-1,8 selon la revendication 1, caractérisé en ce que l'on fait réagir un dérivé de la pipérazine de formule générale : 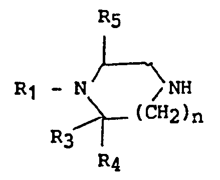 dans laquelle R₁, R₃, R₄, R₅ et n sont définis comme dans la revendication 1, sur une benzo[b]naphtyridine-1,8 de formule générale : 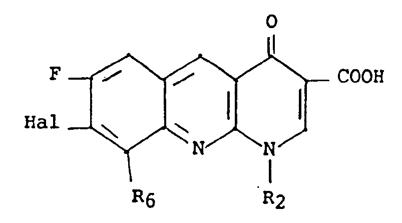 dans laquelle R₂ est défini comme dans la revendication 1 et Hal est un atome de fluor, de chlore ou de brome, si R₆ est un atome d'hydrogène, ou Hal et R₆ sont simultanément des atomes de fluor puis, le cas échéant, lorsque l'on veut obtenir un produit selon la revendication 1 pour lequel R₁ est méthyle et lorsque l'on a obtenu le produit correspondant pour lequel R₁ est un atome d'hydrogène, transforme le produit obtenu en un dérivé de (méthyl-4 pipérazinyl-1)-8 benzo[b]naphtyridine puis éventuellement transforme le produit obtenu en un sel.

9. Procédé de préparation d'un dérivé de la benzo[b]naphtyridine-1,8 selon la revendication 1, caractérisé en ce que l'on transforme l'ester de formule générale : 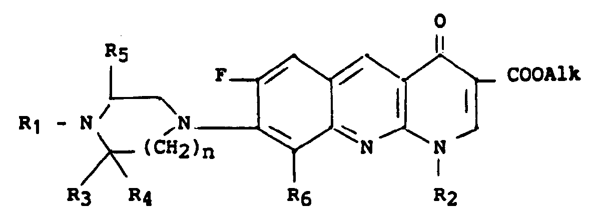 dans laquelle R₁, R₂, R₃, R₄, R₅, R₆ et n sont définis comme dans la revendication 1, ou R₂ représente un radical alcoylamino protégé et Alk est un radical alcoyle droit un ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou lorsque l'on veut préparer un produit selon la revendication 1, pour lequel R₁ est méthyle et lorsque l'on a obtenu le produit correspondant pour lequel R₁ est un atome d'hydrogène, transforme le produit obtenu en un dérivé de (méthyl-4 pipérazinyl-1)-8 benzo[b]naphtyridine puis, éventuellement, prépare le sel du dérivé de benzo[b]naphtyridine obtenu.

10. Composition caractérisée en ce qu'elle contient au moins un dérivé selon la revendication 1, à l'état pur ou en association avec un ou plusieurs diluants ou adjuvants compatibles.

## Revendications (Revendications pour l'(les) Etat(s) contractant(s) suivant(s): ES, GR)

1. Procédé de préparation d'un dérivé de la benzo[b]naphtyridine-1,8 de formule générale : 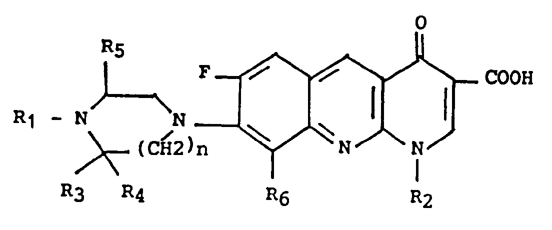 dans laquelle,
soit :
- R₁ représente un atome d'hydrogène ou un radical alcoyle ou hydroxyalcoyle,
- R₂ représente un atome d'hydrogène, un radical alcoyle, fluoroalcoyle, cycloalcoyle, alcoyloxy ou alcoylamino,
- R₃ représente un radical alcoyle et
- R₄ et R₅ sont différents entre eux et représentent un atome d'hydrogène ou un radical alcoyle, ou bien
- R₃ représente un atome d'hydrogène ou un radical alcoyle ou cycloalcoyle et
- R₄ et R₅ sont des atomes d'hydrogène,
- R₆ représente un atome d'hydrogène ou de fluor, et n est égal à 1 ou 2,
les radicaux alcoyle contenant 1 à 4 atomes de carbone en chaîne droite ou ramifiée, les radicaux cycloalcoyle contenant 3 à 6 atomes de carbone, de ses sels métalliques, ses sels d'addition avec les bases azotées et ses sels d'addition avec les acides ainsi que de ses formes hydratées et, le cas échéant de ses isomères et leurs mélanges, caractérisé en ce que l'on fait agir un dérivé de la pipérazine de formule générale : 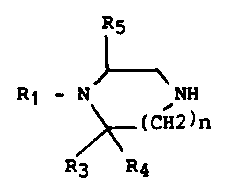 dans laquelle R₁, R₃, R₄ et R₅ sont définis comme ci-dessus sur une benzo[b]naphtyridine-1,8 de formule générale : 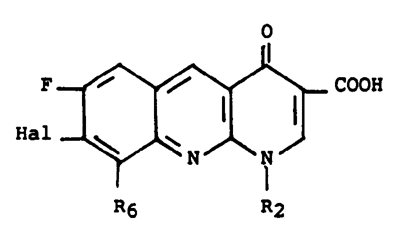 dans laquelle R₂ est défini comme ci-dessus et Hal est un atome de fluor, de chlore ou de brome, si R₆ est un atome d'hydrogène, ou Hal et R₆ sont simultanément des atomes de fluor,
ou bien caractérisé en ce que l'on transforme l'ester de formule générale : 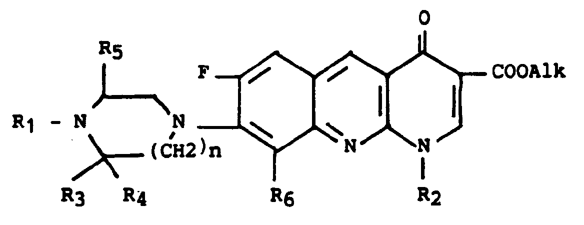 dans laquelle R₁, R₂, R₃, R₄, R₅, R₆ et n sont définis comme ci-dessus ou R₂ représente un radical alcoylamino protégé et Alk est un radical alcoyle droit ou ramifié contenant 1 à 4 atomes de carbone, par toute méthode connue pour obtenir un acide à partir d'un ester, sans toucher au reste de la molécule, puis, le cas échéant, élimine le groupement protecteur du radical alcoylamino, et/ou lorsque l'on veut préparer un produit pour lequel R₁ est méthyle et lorsque l'on a obtenu le produit correspondant pour lequel R₁ est un atome d'hydrogène, transforme le produit obtenu en un dérivé de (méthyl-4 pipérazinyl-1)-8 benzo[b]naphtyridine puis, éventuellement, prépare le sel du dérivé de benzo[b]naphtyridine obtenu.

## Patentansprüche (Patentansprüche für folgende(n) Vertragsstaat(en): AT, BE, CH, DE, DK, FR, GB, IT, LI, LU, NL, SE)

1. Ein 1,8-Benzo[b]naphthyridin-Derivat, dadurch gekennzeichnet, daß es der allgemeinen Formel (I) 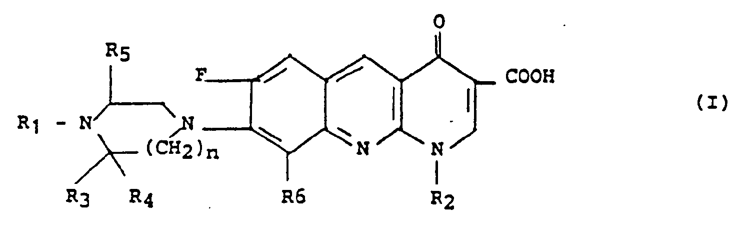 entspricht, in der entweder
- R₁ ein Wasserstoffatom oder einen Alkylrest oder Hydroxyalkylrest darstellt,
- R₂ ein Wasserstoffatom, einen Alkylrest, Fluoralkylrest, Cycloalkylrest, Alkyloxyrest oder Alkylaminorest bedeutet,
- R₃ ein Alkylrest ist,
- R₄ und R₅ untereinander verschieden sind und ein Wasserstoffatom oder einen Alkylrest darstellen, oder
- R₃ ein Wasserstoffatom oder einen Alkylrest oder einen Cycloalkylrest bedeutet, und
- R₄ und R₅ Wasserstoffatome sind,
- R₆ ein Wasserstoffatom oder ein Fluoratom darstellt und
- n gleich 1 oder 2 ist,
wobei die Alkylreste 1 bis 4 Kohlenstoffatome in gerader oder verzweigter Kette und die Cycloalkylreste 3 bis 6 Kohlenstoffatome enthalten, ihre Metallsalze, ihre Additionssalze mit Stickstoffbasen und ihre Additionssalze mit Säuren, sowie ihre hydratisierten Formen und gegebenenfalls ihre Isomeren und ihre Mischungen.

2. Ein Benzonaphthyridin-Derivat nach Anspruch 1, in dem
- R₁ ein Wasserstoffatom oder einen Alkylrest mit 1 oder 2 Kohlenstoffatomen darstellt, gegebenenfalls substituiert durch einen Hydroxyrest.
- R₂ ein Wasserstoffatom oder einen Alkylrest mit 1 bis 4 Kohlenstoffatomen in gerader oder verzweigter Kette bedeutet, gegebenenfalls substituiert durch ein Fluoratom,
- R₃ ein Methylrest ist und
- R₄ und R₅ untereinander verschieden sind und ein Wasserstoffatom oder einen Methylrest bedeuten, oder
- R₃ ein Wasserstoffatom oder ein Methylrest ist und R₄ und R₅ Wasserstoffatome sind,
- R₆ ein Wasserstoffatom oder ein Fluoratom darstellt und
- n 1 oder 2 ist,
ihre Metallsalze, ihre Additionssalze mit Stickstoffbasen und ihre Additionssalze mit Säuren, sowie ihre hydratisierten Formen und gegebenenfalls ihre Isomeren und ihre Mischungen.

3. Ein Derivat nach Anspruch 1, bestehend aus 7-Fluor-1-methyl-8-(3-methyl-1-piperazinyl)-4-oxo-1,4-dihydro-1,8-benzo[b]naphthyridin-3-carbonsäure sowie ihren Salzen.

4. Ein Derivat nach Anspruch 1, bestehend aus 7-Fluor-1-ethyl-8-(1-piperazinyl)-4-oxo-1,4-dihydro-1,8-benzo[b]naphthyridin-3-carbonsäure sowie ihren Salzen.

5. Ein Derivat nach Anspruch 1, bestehend aus 7-Fluor-1-cyclopropyl-8-(1-piperazinyl)-4-oxo-1,4-dihydro-1,8-benzo[b]naphthyridin-3-carbonsäure sowie ihren Salzen.

6. Ein Derivat nach Anspruch 1, bestehend aus 7,9-Difluor-1-methyl-8-(4-methyl-1-piperazinyl)-4-oxo-1,4-dihydro-1,8-benzo[b]naphthyridin-3-carbonsäure sowie ihren Salzen.

7. Ein Derivat nach Anspruch 1, bestehend aus 7-Fluor-1-ethyl-8-(3,4-dimethyl-1-piperazinyl)-4-oxo-1,4-dihydro-1,8-benzo[b]naphthyridin-3-carbonsäure sowie ihren Salzen.

8. Verfahren zur Herstellung eines 1,8-Benzo[b]naphthyridin-Derivates nach Anspruch 1, dadurch gekennzeichnet. daß man ein Piperazin-Derivat der allgemeinen Formel (II) 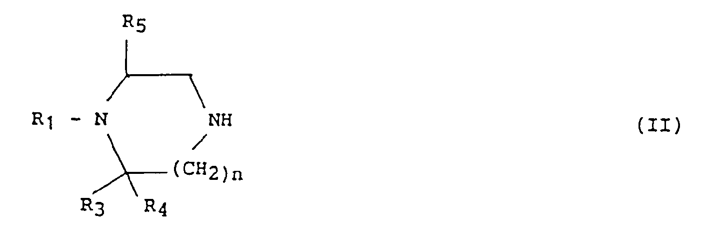 in der R₁, R₃, R₄, R₅ und n wie in Anspruch 1 definiert sind, mit einem 1,8-Benzo[b]naphthyridin der allgemeinen Formel (III) 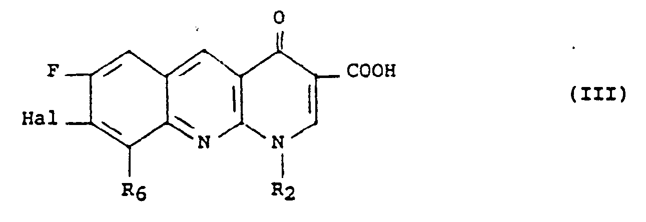 zur Reaktion bringt, in der R₂ wie in Anspruch 1 definiert ist und Hal ein Fluoratom, Chloratom oder Bromatom ist, wenn R₆ Wasserstoff bedeutet, oder Hal und R₆ gleichzeitig Fluoratome darstellen, und man danach, gegebenenfalls, wenn man ein Produkt nach Anspruch 1 erhalten will, in dem R₁ Methyl ist, und wenn man das entsprechende Produkt erhalten hat, in dem R₁ ein Wasserstoffatom ist, dieses erhaltene Produkt in ein 8-(4-Methyl-1-piperazinyl)- benzo[b]naphthyridin umwandelt und anschließend gegebenenfalls das erhaltene Produkt in ein Salz.

9. Verfahren zur Herstellung eines 1,8-Benzo[b]naphthyridin-Derivates nach Anspruch 1, dadurch gekennzeichnet, daß man den Ester der allgemeinen Formel (IV) 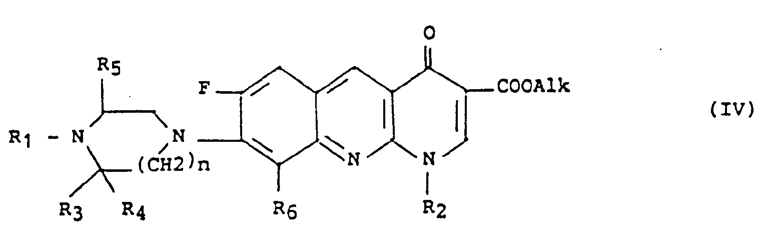 in der R₁, R₂, R₃, R₄, R₅, R₆ und n wie in Anspruch 1 definiert sind. oder R₂ einen geschützten Alkylaminorest darstellt und Alk einen geraden oder verzweigten Alkylrest mit 1 bis 4 Kohlenstoffatomen bedeutet. nach jeder bekannten Methode umwandelt, um eine Säure aus einem Ester zu erhalten. ohne den Rest des Moleküls anzugreifen, und man anschließend gegebenenfalls die Schutzgruppe von dem Alkylaminorest entfernt, und/oder, wenn man ein Produkt nach Anspruch 1 herstellen will, in dem R₁ Methyl ist, und wenn man das entsprechende Produkt erhalten hat, in dem R₁ ein Wasserstoffatom ist, man das erhaltene Produkt in ein 8-(4-Methyl-1-piperazinyl)-benzo[b]naphthyridin umwandelt und anschließend gegebenenfalls das Salz des erhaltenen Benzo[b]naphthyridin-Derivates herstellt.

10. Zusammensetzung. dadurch gekennzeichnet, daß sie mindestens ein Derivat nach Anspruch 1, im reinen Zustand oder in Assoziation mit einem oder mehreren kompatiblen Verdünnungsmitteln oder Zusatzstoffen enthält.

## Patentansprüche (Patentansprüche für folgende(n) Vertragsstaat(en): ES, GR)

1. Verfahren zur Herstellung eines 1,8-Benzo[b]naphthyridin-Derivates der allgemeinen Formel (I) 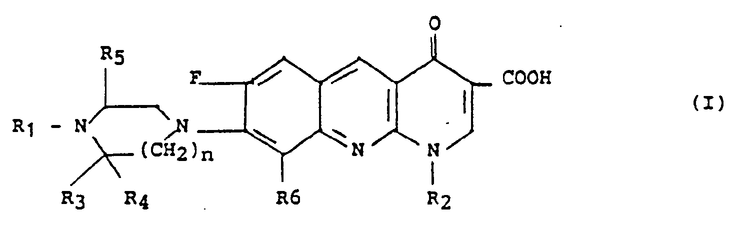 in der entweder
- R₁ ein Wasserstoffatom oder einen Alkylrest oder Hydroxyalkylrest darstellt,
- R₂ ein Wasserstoffatom, einen Alkylrest, Fluoralkylrest, Cycloalkylrest, Alkyloxyrest oder Alkylaminorest bedeutet,
- R₃ ein Alkylrest ist,
- R₄ und R₅ untereinander verschieden sind und ein Wasserstoffatom oder einen Alkylrest darstellen, oder
- R₃ ein Wasserstoffatom oder einen Alkylrest oder einen Cycloalkylrest bedeutet, und
- R₄ und R₅ Wasserstoffatome sind,
- R₆ ein Wasserstoffatom oder ein Fluoratom darstellt und
- n gleich 1 oder 2 ist,
wobei die Alkylreste 1 bis 4 Kohlenstoffatome in gerader oder verzweigter Kette und die Cycloalkylreste 3 bis 6 Kohlenstoffatome enthalten, ihren Metallsalzen, ihren Additionssalzen mit Stickstoffbasen und ihren Additionssalzen mit Säuren, sowie ihren hydratisierten Formen und gegebenenfalls ihren Isomeren und ihren Mischungen dadurch gekennzeichnet, daß man ein Piperazin-Derivat der allgemeinen Formel (II) 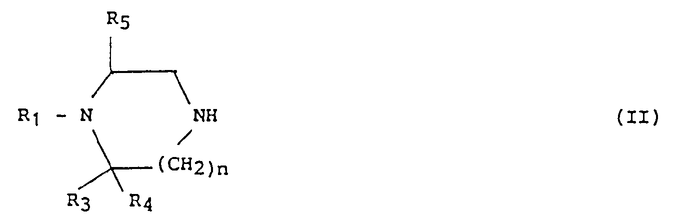 in der R₁, R₃, R₄, R₅ und n wie vorstehend definiert sind, mit einem 1,8-Benzo[b]naphthyridin der allgemeinen Formel (III) 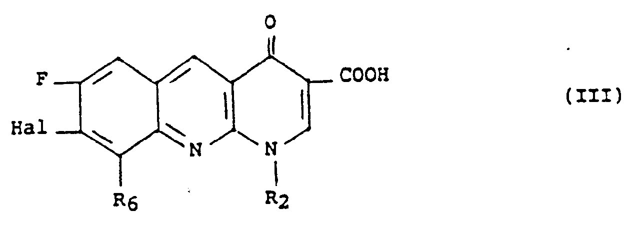 zur Reaktion bringt. in der R₂ wie vorstehend definiert ist und Hal ein Fluoratom, Chloratom oder Bromatom ist, wenn R₆ Wasserstoff bedeutet, oder Hal und R₆ gleichzeitig Fluoratome darstellen,
oder dadurch gekennzeichnet, daß man den Ester der allgemeinen Formel (IV) 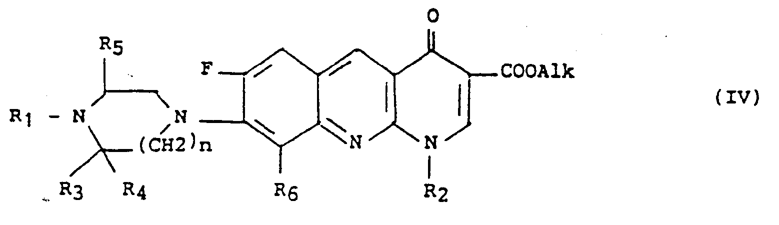 in der R₁, R₂, R₃, R₄, R₅, R₆ und n wie vorstehend definiert sind, oder R₂ einen geschützten Alkylaminorest darstellt und Alk einen geraden oder verzweigten Alkylrest mit 1 bis 4 Kohlenstoffatomen bedeutet, nach jeder bekannten Methode umwandelt, um eine Säure aus einem Ester zu erhalten, ohne den Rest des Moleküls anzugreifen, und man anschließend gegebenenfalls die Schutzgruppe von dem Alkylaminorest entfernt, und/oder, wenn man ein Produkt herstellen will. in dem R₁ Methyl ist. und wenn man das entsprechende Produkt erhalten hat, in dem R₁ ein Wasserstoffatom ist, man das erhaltene Produkt in ein 8-(4-Methyl-1-piperazinyl)-benzo[b]naphthyridin umwandelt und anschließend gegebenenfalls das Salz des erhaltenen Benzo[b]naphthyridin-Derivates herstellt.

## Claims (Claims for the following Contracting State(s): AT, BE, CH, DE, DK, FR, GB, IT, LI, LU, NL, SE)

1. A benzo[b][1,8]naphthyridine derivative, characterised in that it corresponds to the general formula: 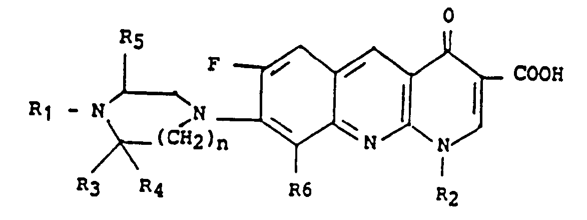 in which,
either:
- R₁ represents a hydrogen atom or an alkyl or hydroxyalkyl radical,
- R₂ represents a hydrogen atom or an alkyl, fluoroalkyl, cycloalkyl, alkyloxy or alkylamino radical,
- R₃ represents an alkyl radical and
- R₄ and R₅ are different from each other and represent a hydrogen atom or an alkyl radical, or else
- R₃ represents a hydrogen atom or an alkyl or cycloalkyl radical and
- R₄ and R₅ are hydrogen atoms
- R₆ represents a hydrogen or fluorine atom, and n is equal to 1 or 2,
the alkyl radicals containing 1 to 4 straight- or branched-chain carbon atoms and the cycloalkyl radicals containing 3 to 6 carbon atoms,
its metal salts, its addition salts with nitrogenous bases and its addition salts with acids, as well as its hydrated forms and, if appropriate, its isomers and their mixtures.

2. A benzonaphthyridine derivative according to Claim 1, for which
- R₁ represents a hydrogen atom or an alkyl radical containing 1 or 2 carbon atoms, optionally substituted by a hydroxyl radical,
- R₂ represents a hydrogen atom or an alkyl radical containing 1 to 4 straight- or branched-chain carbon atoms, optionally substituted by a fluorine atom,
- R₃ represents a methyl radical and
- R₄ and R₅, which are different from each other, represent a hydrogen atom or a methyl radical, or else
- R₃ is a hydrogen atom or a methyl radical and R₄ and R₅ represent hydrogen atoms,
- R₆ represents a hydrogen or fluorine atom and
- n is equal to 1 or 2,
its metal salts, its addition salts with nitrogenous bases and its addition salts with acids, as well as its hydrated forms and, if appropriate, its isomers and their mixtures.

3. A derivative according to Claim 1, consisting of 7-fluoro-1-methyl-8-(3-methyl-1-piperazinyl)-4-oxo- 1,4-dihydrobenzo[b][1,8]naphthyridine-3-carboxylic acid as well as its salts.

4. A derivative according to Claim 1, consisting of 1-ethyl-7-fluoro-8-(1-piperazinyl)-4-oxo-1,4-dihydro-benzo[b][1,8]naphthyridine-3-carboxylic acid as well as its salts.

5. A derivative according to Claim 1, consisting of 1-cyclopropyl-7-fluoro-8-(1-piperazinyl)-4-oxo- 1, 4-dihydrobenzo[b][1,8]naphthyridine-3-carboxylic acid as well as its salts.

6. A derivative according to Claim 1, consisting of 7,9-difluoro-1-methyl-8,(4-methyl-1-piperazinyl)-4-oxo- 1,4-dihydrobenzo[b][1,8]naphthyridine-3-carboxylic acid as well as its salts.

7. A derivative according to Claim 1, consisting of 8-(3,4-dimethyl-1-piperazinyl)-1-ethyl-7-fluoro-4-oxo- 1,4-dihydrobenzo[b][1,8]naphthyridine-3-carboxylic acid as well as its salts.

8. Process for the preparation of a benzo[b][1,8]- naphthyridine derivative according to Claim 1, characterised in that a piperazine derivative of general formula: 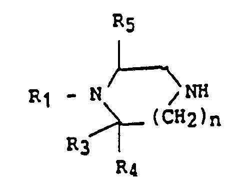 in which R₁, R₃, R₄, R₅ and n are defined as in Claim 1, is reacted with a benzo[b][1,8]naphthyridine of general formula: 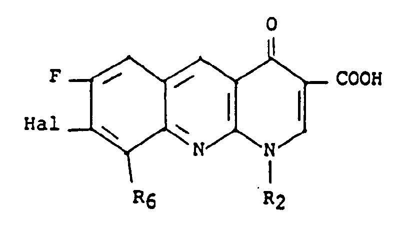 in which R₂ is defined as in Claim 1 and Hal is a fluorine, chlorine or bromine atom, if R₆ is a hydrogen atom, or Hal and R₆ are simultaneously fluorine atoms, then, if appropriate, when it is desired to obtain a product according to Claim 1 for which R₁ is methyl and when the corresponding product has been obtained for which R₁ is a hydrogen atom, the product obtained is converted to an 8-(4-methyl-1-piperazinyl)benzo[b]- naphthyridine derivative and then the product obtained is optionally converted to a salt.

9. Process for the preparation of a benzo[b][1,8]- naphthyridine derivative according to Claim 1, characterised in that the ester of general formula: 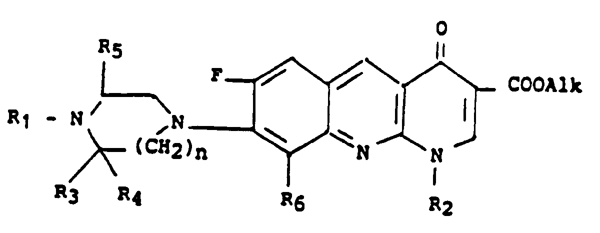 in which R₁, R₂, R₃, R₄, R₅, R₆ and n are defined as in Claim 1, or R₂ represents a protected alkylamino radical and Alk is a straight or branched alkyl radical containing 1 to 4 carbon atoms, is converted by any method known for producing an acid from an ester, without affecting the remainder of the molecule, then, if appropriate, the protective group of the alkylamino radical is removed, and/or, when it is desired to prepare a product according to Claim 1 for which R₁ is methyl and when the corresponding product has been obtained for which R₁ is a hydrogen atom, the product obtained is converted to an 8-(4-methyl-1-piperazinyl)benzo[b]naphthyridine derivative, and then, optionally, the salt of the benzo[b]-naphthyridine derivative obtained is prepared.

10. Composition, characterised in that it contains at least one derivative according to Claim 1 in the pure form or in combination with one or a number of compatible diluents or adjuvants.

## Claims (Claims for the following Contracting State(s): ES, GR)

1. Process for the preparation of a benzo[b][1,8]-naphthyridine derivative of general formula: 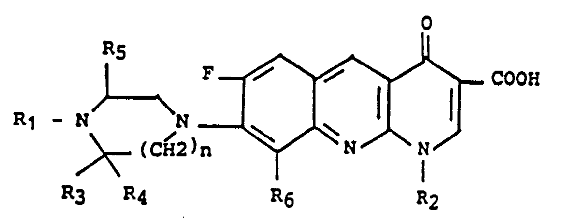 in which,
either:
- R₁ represents a hydrogen atom or an alkyl or hydroxyalkyl radical,
- R₂ represents a hydrogen atom or an alkyl, fluoroalkyl, cycloalkyl, alkyloxy or alkylamino radical,
- R₃ represents an alkyl radical and
- R₄ and R₅ are different from each other and represent a hydrogen atom or an alkyl radical, or else
- R₃ represents a hydrogen atom or an alkyl or cycloalkyl radical and
- R₄ and R₅ are hydrogen atoms
- R₆ represents a hydrogen or fluorine atom, and n is equal to 1 or 2,
the alkyl radicals containing 1 to 4 straight- or branched-chain carbon atoms and the cycloalkyl radicals containing 3 to 6 carbon atoms,
its metal salts, its addition salts with nitrogenous bases and its addition salts with acids, as well as its hydrated forms and, if appropriate, its isomers and their mixtures, characterised in that a piperazine derivative of general formula 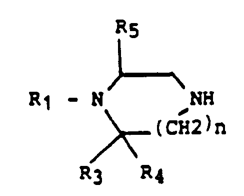 in which R₁, R₃, R₄ and R₅ are defined as above, is reacted with a benzo[b][1,8]naphthyridine of general formula: 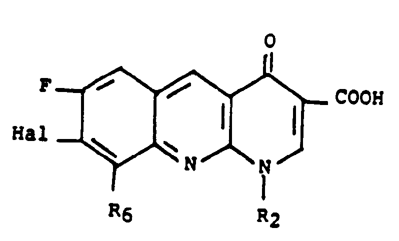 in which R₂ is defined as above and Hal is a fluorine, chlorine or bromine atom, if R₆ is a hydrogen atom, or Hal and R₆ are simultaneously fluorine atoms,
or else characterised in that the ester of general formula: 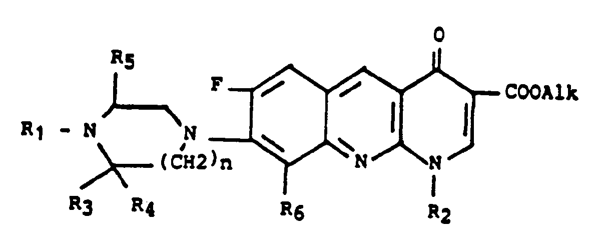 in which R₁, R₂, R₃, R₄, R₅, R₆ and n are defined as above, or R₂ represents a protected alkylamino radical and Alk is a straight or branched alkyl radical containing 1 to 4 carbon atoms, is converted by any method known for producing an acid from an ester, without affecting the remainder of the molecule, then, if appropriate, the protective group of the alkylamino radical is removed, and/or, when it is desired to prepare a product for which R₁ is methyl and when the corresponding product has been obtained for which R₁ is a hydrogen atom, the product obtained is converted to an 8-(4-methyl-1-piperazinyl)-benzo[b]naphthyridine derivative, and then, optionally, the salt of the benzo[b]naphthyridine derivative obtained is prepared.
